(19) Europäisches Patentamt / European Patent Office / Office européen des brevets

(11) **EP 2 762 064 B1**

(12) **FASCICULE DE BREVET EUROPEEN**

(45) Date de publication et mention
de la délivrance du brevet:
**07.06.2023 Bulletin 2023/23**

(21) Numéro de dépôt: **14153554.2**

(22) Date de dépôt: **31.01.2014**

(51) Classification Internationale des Brevets (IPC):
**A61B 5/00** *(2006.01)*

(52) Classification Coopérative des Brevets (CPC):
**A61B 5/0075; A61B 5/411; A61B 5/445;
G16C 99/00**

(54) **Procédé et système de calcul d'un indicateur de quantification d'une réaction dermique de la peau d'un être vivant**

Berechnungsverfahren und -system eines Indikators zur Quantisierung einer Hautreaktion eines Lebewesens

Method and system for calculating an indicator for quantifying a dermal reaction of the skin of a living being

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.02.2013 FR 1350905**

(43) Date de publication de la demande:
**06.08.2014 Bulletin 2014/32**

(73) Titulaire: **Commissariat à l'Énergie Atomique
et aux Énergies Alternatives
75015 Paris (FR)**

(72) Inventeur: **Koenig, Anne
38410 Saint Martin d'Uriage (FR)**

(74) Mandataire: **Lavoix
2, place d'Estienne d'Orves
75441 Paris Cedex 09 (FR)**

(56) Documents cités:
**US-A1- 2010 160 754     US-A1- 2011 124 987
US-A1- 2012 130 257**

• **NIKIFOROS KOLLIAS ET AL: "Interpreting diffuse reflectance for in vivo skin reactions in terms of chromophores", JOURNAL OF BIOPHOTONICS, vol. 3, no. 1-2, 27 janvier 2010 (2010-01-27), pages 15-24, XP055090830, ISSN: 1864-063X, DOI: 10.1002/jbio.200900066**
• **ANNE KOENIG ET AL: "Diffuse reflectance spectroscopy: a clinical study of tuberculin skin tests reading", PROCEEDINGS OF SPIE, vol. 8592, 21 février 2013 (2013-02-21), pages 85920S-85920S-8, XP055090850, ISSN: 0277-786X, DOI: 10.1117/12.2002314**

**Description**

La présente invention concerne un procédé de calcul d'un indicateur de quantification d'une réaction dermique de la peau d'un être vivant, telle qu'une réaction dermique suite à l'injection d'un principe actif. Par la suite, la réaction dermique de la peau est également appelée réaction cutanée.

Le procédé de calcul comprend les étapes suivantes :

- l'éclairement d'une zone à caractériser de la peau via un faisceau lumineux d'excitation émis par une source de lumière, la zone à caractériser comportant la réaction dermique,
- la mesure, à l'aide d'un spectromètre, du spectre d'un rayonnement rétrodiffusé issu de la peau suite à l'éclairement de ladite zone à caractériser,
- la détermination, à partir du spectre mesuré et pour au moins une valeur donnée de la longueur d'onde du faisceau lumineux, d'une valeur du coefficient d'absorption de la zone à caractériser,
- le calcul, à partir de la ou chaque valeur déterminée du coefficient d'absorption, de la concentration d'au moins un chromophore de la peau, et
- le calcul de l'indicateur de quantification de la réaction dermique en fonction de la concentration du ou de chaque chromophore précédemment calculée,

L'invention concerne également un système de calcul d'un tel indicateur de quantification.

L'invention s'applique en particulier au calcul d'un indicateur de quantification de la réaction dermique d'un patient suite à l'injection intradermique d'un principe actif pour la mise en oeuvre d'un test de la présence d'anticorps dans l'organisme, ce test étant par exemple le test de la tuberculose suite à une injection de tuberculine. L'indicateur de quantification est alors fonction de la réponse immunitaire.

L'invention s'applique également au calcul d'un indicateur de quantification de la réaction cutanée suite à l'injection d'un principe actif apte à entraîner une allergie cutanée.

L'invention s'applique plus généralement au calcul d'un indicateur de quantification de toute réaction dermique, tel qu'une réaction d'inflammation, une réaction d'induration, encore une réaction d'altération de la peau.

US 2011/124987 A1 décrit un procédé de calcul d'un indicateur de quantification d'une réaction dermique de la peau d'un être vivant. US 2012/130257 A1 et US 2010/160754 A1 décrivent chacun un procédé dans lequel des coefficients d'absorption $\mu_a$ et de diffusion $\mu_s$ sont déterminés à partir du spectre réfléchi.

L'article « Interpreting diffuse réflectance for in vivo skin reactions in terms of chromophores » de Kollias et al, publié dans la revue intitulée Journal of Biophotonics en 2010, décrit un procédé et un système de calcul du type précité. Cet article est une interprétation quantitative de réactions cutanées à partir de mesures d'une réflectance de la peau, en formant notamment l'hypothèse que l'atténuation de la lumière, durant sa propagation à travers la peau, suit la loi de Beer-Lambert.

Le mode opératoire est le suivant. La peau est illuminée avec un faisceau lumineux issu d'une source de lumière blanche, des mesures de réflectance d'un rayonnement rétrodiffusé dû à l'éclairement de la peau sont alors effectuées à l'aide d'un spectromètre. Les concentrations en chromophores de la peau, notamment les concentrations en mélanine, en oxyhémoglobine et en désoxyhémoglobine, sont calculées à partir des valeurs de réflectance mesurées.

L'absorbance de la peau est considérée comme dépendante de concentrations en chromophores calculées en fonction des valeurs de réflectance mesurées. Dans le cas de la formation de pigments induite par une radiation solaire simulée, la seule concentration en chromophore prise en compte pour la quantification est la concentration en mélanine. Dans le cas d'une inflammation induite par l'histamine, les concentrations en chromophores prises en compte sont les concentrations en en oxyhémoglobine $HbO_2$ et en eau. L'effet de la diffusion est approximé par la prise en compte d'un chromophore supplémentaire. Ainsi, on assimile la diffusion à une absorption liée à la concentration de ce nouveau chromophore.

Enfin, dans ce document, les auteurs établissent l'évolution de ces chromophores en fonction de certaines inflammations. Cependant, ils ne calculent pas d'indice permettant de quantifier la réaction de la peau à partir de ces concentrations. De l'aveu même des auteurs, les relations entre les concentrations des différents chromophores n'ont pas été explorées de manière exhaustive, et demeurent non-établies.

Les inventeurs ont de leur côté constaté qu'il était nécessaire d'établir un indicateur multiparamétrique, c'est-à-dire tenant compte de la concentration de différents chromophores, permettant de quantifier la réaction de la peau suite à l'injection du principe actif. De plus, ils ont observé qu'une telle quantification, réalisée à l'aide des concentrations de différents chromophores n'est pas toujours très précise, et un certain nombre de résultats sont erronés, la réaction étant par exemple indiquée à tort comme étant positive (faux positif), ou au contraire comme étant négative alors qu'elle est en réalité positive (faux négatif).

Le but de l'invention est donc de proposer un procédé et un système de calcul permettant d'améliorer la pertinence de l'indicateur de quantification calculé, tout en permettant de calculer cet indicateur à plus bref délai après la stimulation à l'origine de la réaction dermique.

A cet effet, l'invention a pour objet un procédé de calcul selon la revendication 1 ou selon la revendication 2.

Suivant d'autres aspects avantageux de l'invention, le procédé de calcul est selon l'une quelconque des revendications 3 à 10.

L'invention a également pour objet un système de calcul selon la revendication 11 ou selon la revendication 12.

Ces caractéristiques et avantages de l'invention apparaîtront à la lecture de la description qui va suivre, donnée uniquement à titre d'exemple non limitatif, et faite en référence aux dessins annexés, sur lesquels :

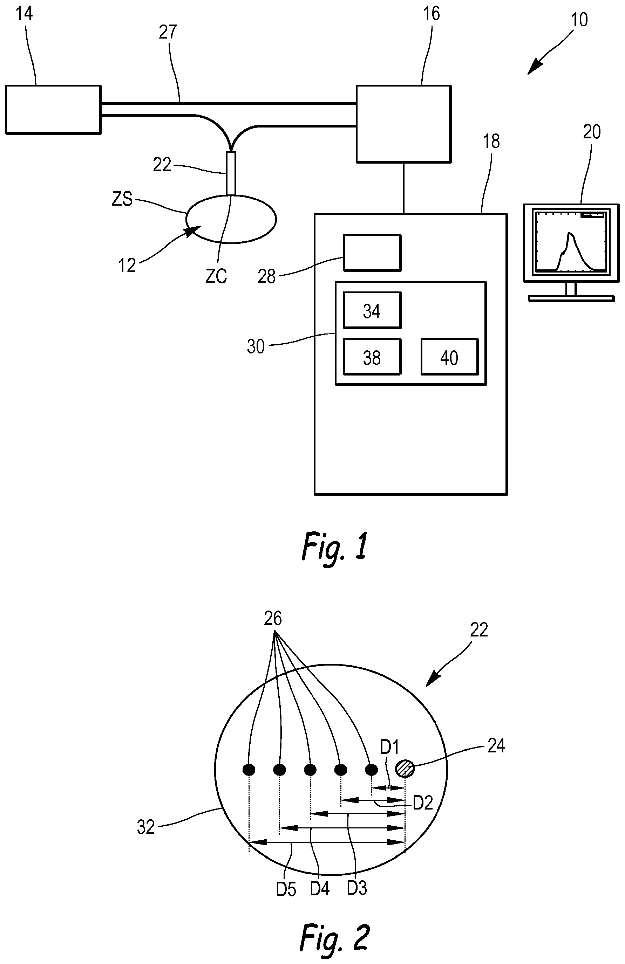

- la figure 1 est une représentation très schématique d'un système de calcul d'un indicateur de quantification d'une réaction dermique, le système de calcul comprenant une source de lumière propre à éclairer une zone de la peau, un spectromètre propre à mesurer le spectre d'un rayonnement rétrodiffusé issu de la peau suite à l'éclairement de ladite zone, et une unité de traitement d'informations,
- la figure 2 est une représentation schématique d'une extrémité d'une sonde destinée à être au contact de la peau, la sonde comportant une fibre optique d'excitation pour véhiculer le faisceau lumineux issu de la source de lumière et une pluralité de fibres optiques de détection pour véhiculer jusqu'au spectromètre le rayonnement rétrodiffusé par la peau,

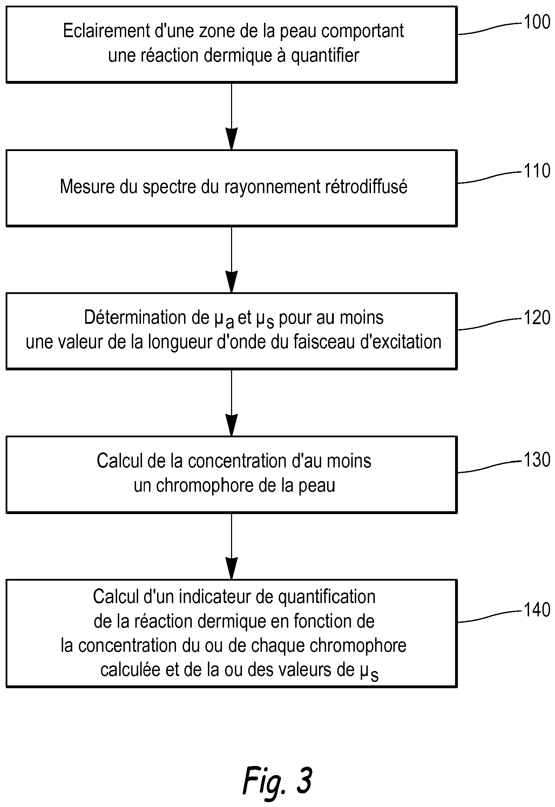

- la figure 3 est un organigramme d'un procédé de calcul selon l'invention,

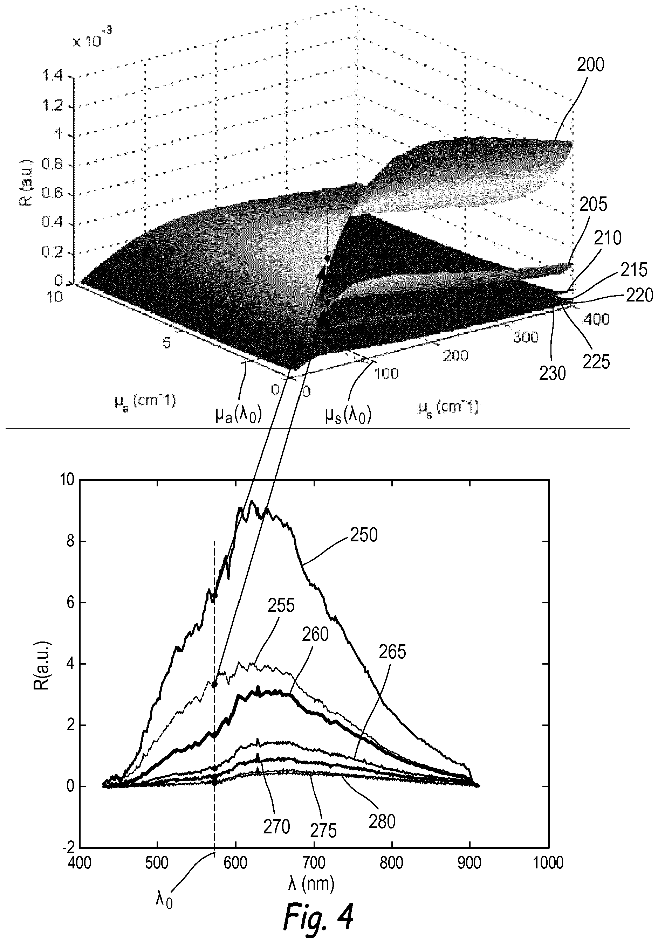

- la figure 4 est un ensemble de surfaces représentant la réflectance en fonction du coefficient d'absorption et du coefficient de diffusion, chaque surface correspondant à une distance respective entre la fibre d'excitation et la fibre de détection correspondante, et un ensemble de courbes représentant la réflectance en fonction de la longueur d'onde du faisceau lumineux incident, chaque courbe correspondant à ladite distance respective,

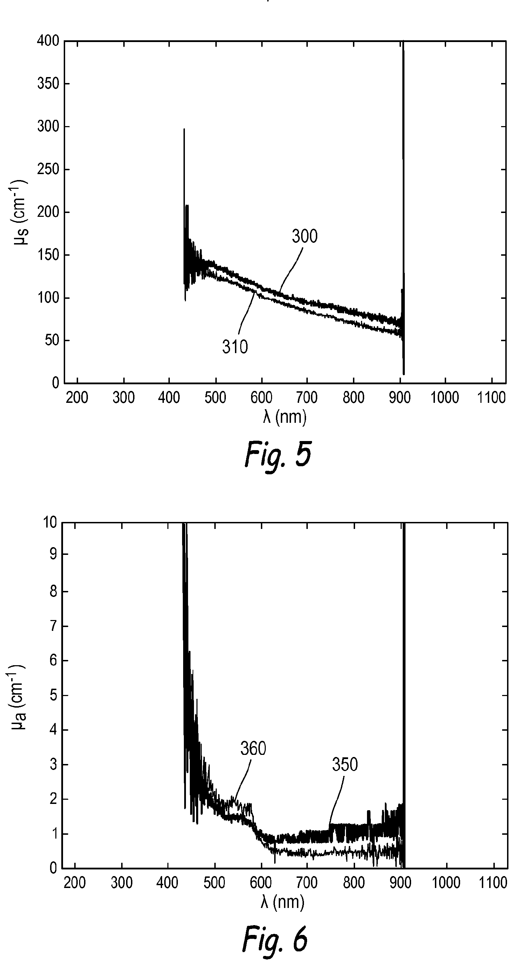

- la figure 5 est un ensemble de courbes représentant le coefficient de diffusion en fonction de la longueur d'onde du faisceau lumineux incident, pour une zone saine de la peau, et respectivement pour une zone à caractériser comportant une réaction dermique,
- la figure 6 est un ensemble de courbes représentant le coefficient d'absorption en fonction de la longueur d'onde du faisceau lumineux incident, pour une zone saine de la peau, et respectivement pour une zone à caractériser comportant une réaction dermique,

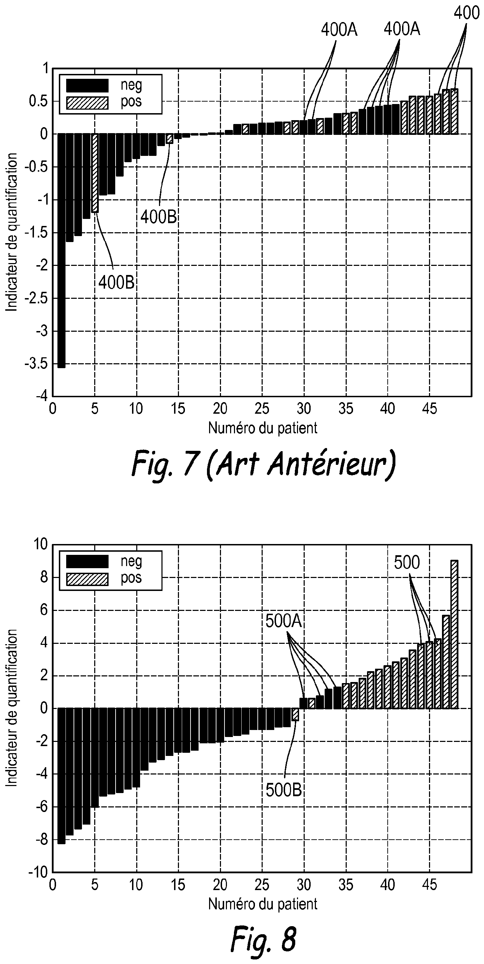

- la figure 7 est un histogramme représentant des valeurs de l'indicateur de quantification calculées pour une pluralité de patients en mettant en oeuvre un procédé de l'état de la technique, et

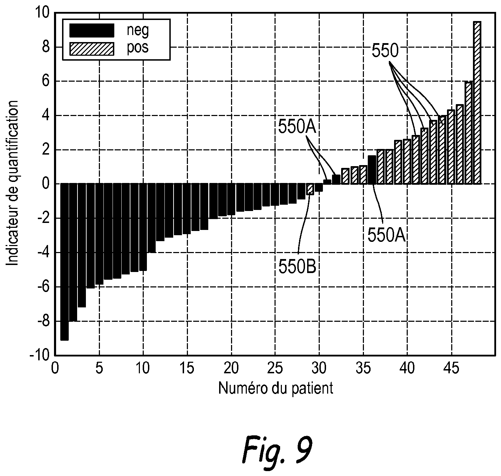

- les figures 8 et 9 sont des vues analogues à celle de la figure 7 en mettant en oeuvre un procédé selon un premier mode de réalisation, et respectivement selon un deuxième mode de réalisation de l'invention ; le premier mode de réalisation n'étant pas couvert par le texte des revendications, mais étant considéré comme utile à la compréhension de l'invention.

Sur la figure 1, un système de calcul 10 est destiné à calculer un indicateur IND de quantification d'une réaction dermique de la peau 12 d'un être vivant, telle qu'une réaction dermique suite à l'injection intradermique d'un principe actif pour la mise en oeuvre d'un test de la présence d'anticorps dans l'organisme. La réaction dermique de la peau est également appelée réaction cutanée. Le principe actif est, par exemple, la tuberculine pour la mise en oeuvre du test de la tuberculose. L'indicateur de quantification IND est alors fonction de la réponse immunitaire.

Ainsi, de façon générale, le système de calcul 10 est destiné à caractériser, via le calcul de l'indicateur de quantification IND, toute réaction dermique, en particulier une réaction de coloration, une réaction d'inflammation, une réaction d'induration, encore une réaction d'altération de la peau.

Le système de calcul 10 comprend une source de lumière 14 propre à émettre un faisceau lumineux pour éclairer une zone à caractériser ZC de la peau 12, ladite zone à caractériser ZC comportant la réaction dermique, et en complément propre à éclairer une zone saine ZS de la peau.

Le système de calcul 10 comprend un spectromètre 16 propre à mesurer le spectre M d'un rayonnement rétrodiffusé issu de la peau 12 suite à l'éclairement de ladite zone à caractériser. Par rayonnement rétrodiffusé, on entend le rayonnement passant à travers la peau 12 depuis une zone source correspondant à la zone de l'épiderme éclairée par le faisceau lumineux issu de la source de lumière 14, et détecté dans une zone de détection à une distance donnée de la zone source, les trajets les plus probables des photons détectés à ladite distance donnée de la zone source étant en forme d'une banane. L'homme du métier observera alors que plus la zone de détection est éloignée de la zone source, plus les photons auront voyagé profondément dans la peau 12, et moins ils seront nombreux à ressortir à cet endroit compte tenu de l'absorption des photons par la peau 12.

Le système de calcul 10 comprend une unité de traitement d'informations 18 et un écran 20 d'affichage de différents indicateurs de quantification IND calculés pour différents êtres vivants, ces indicateurs calculés étant par exemple représentés sous la forme d'histogrammes, visibles sur les figures 8 et 9.

En complément, le système de calcul 10 comprend une sonde 22 destinée à être positionnée au contact de la peau 12 et comportant, comme représenté sur la figure 2, au moins une fibre optique d'excitation 24 pour véhiculer le faisceau lumineux depuis la source 14 jusqu'à la peau 12, et une pluralité de fibres optiques de détection 26, chaque fibre optique de détection 26 étant propre à véhiculer jusqu'au spectromètre 16 une partie du rayonnement rétrodiffusé par la peau 12. Avantageusement, les fibres de détection 26 sont agencées selon des couronnes concentriques et la fibre d'excitation 24 est disposée au centre desdites couronnes. Ainsi, on dispose de plusieurs fibres de détection 26 pour une même distance Di avec la fibre d'excitation 24. Les signaux mesurés pour une même distance Di sont alors moyennés, ce qui permet d'augmenter le rapport signal sur bruit.

La peau 12 comporte, comme connu en soi, une pluralité de chromophores, notamment la mélanine, l'oxyhémoglobine, également notée $HbO_2$, la désoxyhémoglobine, également notée Hb, l'eau et la bilirubine.

La peau 12 est, par exemple, la peau d'un patient, comme cela sera décrit par la suite dans l'exemple des figures 8 et 9. En variante, la peau 12 est la peau d'un animal, tel qu'un mammifère, des tests ayant été également effectués à titre d'exemple sur la peau d'un cochon.

La source de lumière 14, visible sur la figure 1, est propre à émettre un faisceau lumineux de longueur d'onde $\lambda$, les valeurs de la longueur d'onde $\lambda$ étant de préférence comprises entre 400 nm et 1000 nm. Autrement dit, la source de lumière 14 est propre à émettre un faisceau lumineux en lumière blanche et en proche infrarouge.

En complément, dans l'exemple de la figure 1, la source de lumière 14 est aussi reliée directement au spectromètre 16 par l'intermédiaire d'une fibre optique de liaison directe 27, également appelée retour d'excitation. Cette configuration, optionnelle, permet une prise en compte plus précise du spectre d'émission, ainsi que de ses éventuelles fluctuations, et ainsi d'améliorer la fiabilité du résultat.

Le spectromètre 16 est couplé aux fibres optiques de détection 26, par exemple par l'intermédiaire d'une platine de translation, non représentée, permettant le multiplexage de toutes les fibres de détection 26 sur un seul spectromètre. Le spectromètre 16 est, par exemple, un spectromètre à fibre. En variante, un spectromètre multivoies est utilisé. D'une façon générale, le spectromètre 16 est apte à produire un signal sur différentes plages de longueur d'onde déterminées. Il s'agit par exemple d'une série de détecteurs optiques, tels que des photodiodes, chaque détecteur étant associé une plage de longueur d'onde déterminée.

L'unité de traitement d'informations 18 comporte un processeur de données 28 et une mémoire 30 associée au processeur.

La sonde 22 comporte un boitier 32 de protection des fibres optiques 24, 26, le diamètre extérieur du boîtier de protection 30 étant par exemple de l'ordre de 6 mm. La sonde 22 comporte, par exemple, une unique fibre d'excitation 24 et quarante-deux fibres de détection 26. Plus précisément, les fibres de détection 26 sont agencées en 6 couronnes concentriques, chaque couronne comportant 7 fibres.

La fibre d'excitation 24 présente, par exemple, un diamètre de l'ordre de 500 $\mu$m, et chaque fibre de détection 26 présente, par exemple, un diamètre de l'ordre de 300 $\mu$m.

Les fibres optiques de détection 26 sont de préférence positionnées à différentes distances $D_i$ de la fibre optique d'excitation 24, où i est l'indice de la distance, i étant un nombre entier variant entre 1 et N, avec N le nombre de distances prédéterminées distinctes. A titre d'exemple sur la figure 2, la sonde 22 comporte cinq fibres de détection 26 positionnées respectivement à des distances $D_1$, $D_2$, $D_3$, $D_4$ et $D_5$. Par convention, on considérera que plus l'indice i est petit, plus la distance $D_i$ associée est faible.

En complément non représenté, les quarante-deux fibres de détection 26 sont réparties en six groupes de sept fibres de détection 26 positionnées en six distances respectives $D_1$, $D_2$, $D_3$, $D_4$, $D_5$ et $D_6$ de la fibre d'excitation 24. Toutes les fibres de détection 26 d'un même groupe sont positionnées à une même distance $D_i$ de la fibre d'excitation 24.

Le faisceau lumineux propre à transiter par le retour d'excitation 27 pour être reçu par le spectromètre 16 est un faisceau présentant une intensité de valeur atténuée par rapport à celle du rayonnement incident à la peau, afin d'éviter un éblouissement du spectromètre 16. Dans l'exemple décrit, un faisceau d'excitation est formé de 18 fibres optiques d'excitation 24, tandis que le retour d'excitation 27 est formé d'une seule fibre. Ainsi, le signal de retour d'excitation adressé au spectromètre 16 a une intensité atténuée par rapport au signal d'excitation, l'atténuation résultant du fait que le retour d'excitation 27 est formé d'une unique fibre optique alors que 18 fibres d'excitation 24 constituent le faisceau d'excitation.

La mémoire 30 est apte à stocker un premier logiciel 34 de détermination, à partir du spectre mesuré M et pour au moins une valeur donnée de la longueur d'onde $\lambda$ du faisceau lumineux, d'une valeur du coefficient d'absorption $\mu_a$ de la zone correspondante ZC, ZS de la peau 12. Le premier logiciel 34 de détermination est également apte à déterminer, à partir du spectre mesuré M et pour au moins une valeur donnée de la longueur d'onde $\lambda$ du faisceau lumineux, une valeur du coefficient de diffusion $\mu_s$ de la zone correspondante ZC, ZS de la peau 12.

Les inventeurs ont estimé qu'il était important de considérer le coefficient d'absorption $\mu_a$ et le coefficient de diffusion $\mu_s$ comme des grandeurs de natures différentes, la diffusion n'étant pas assimilable à une absorption. En effet, contrairement à l'état de la technique, il est imprécis d'assimiler le coefficient de diffusion $\mu_s$ à un chromophore, dont la concentration est déterminée à partir de la valeur de l'atténuation. Autrement dit, la diffusion n'est pas une composante de l'absorption. La prise en compte rigoureuse de la diffusion dans un milieu diffusant suppose au contraire de considérer l'absorption et la diffusion comme des phénomènes différents. Il est alors possible d'estimer simultanément une valeur du coefficient d'absorption $\mu_a$ et une valeur du coefficient de diffusion $\mu_s$ pour caractériser la propagation de la lumière dans le milieu, ces estimations étant réalisées à partir de la mesure de la lumière rétrodiffusée par le tissu.

Le terme coefficient de diffusion représente indifféremment le coefficient de diffusion réduit, noté $\mu_s'$, que le coefficient de diffusion, noté $\mu_s$, sachant que ces deux grandeurs sont liées par la relation :

$$\mu_s' = \mu_s \times (1\text{-}g)$$

g désignant le coefficient d'anisotropie, ce dernier étant supposé constant et égal à 0,8 dans les tissus biologiques.

Dans la suite du texte, ces grandeurs sont désignées par le terme coefficient de diffusion, en utilisant la notation $\mu_s$.

La mémoire 30 est apte à stocker un premier logiciel 38 de calcul, à partir de la ou chaque valeur déterminée du coefficient d'absorption $\mu_a$, la concentration d'au moins un chromophore de la peau 12.

La mémoire 30 est également apte à stocker un deuxième logiciel 40 de calcul de l'indicateur IND de quantification de la réaction dermique en fonction de la concentration du ou de chaque chromophore précédemment calculée et en fonction également de la ou chaque valeur déterminée du coefficient de diffusion $\mu_s$.

En variante, le deuxième logiciel de calcul 40 est propre à calculer l'indicateur de quantification IND en fonction d'un écart entre les concentrations du ou de chaque chromophore Hb, $HbO_2$ de la zone saine ZS et de la zone à caractériser ZC et en fonction du ou des écarts entre les valeurs déterminées du coefficient de diffusion $\mu_s$ de la zone saine ZS et de la zone à caractériser ZC.

Le fonctionnement du système de calcul 10 selon l'invention va désormais être décrit à l'aide de l'organigramme de la figure 3 illustrant un procédé de calcul selon l'invention de l'indicateur IND de quantification de la réaction cutanée.

Lors de l'étape initiale 100, la zone correspondante ZC, ZS de la peau 12 est tout d'abord éclairée à l'aide de la source de lumière 14 pour une valeur donnée de la longueur d'onde $\lambda$ du faisceau lumineux. Pour ce faire, un opérateur place la sonde 22 en contact avec la zone correspondante ZC, ZS de la peau 12, de manière à ce que la fibre d'excitation 24 et les fibres de détection 26 soient en regard de ladite zone correspondante ZC, ZS.

Le spectre M du rayonnement rétrodiffusé issu de la peau 12 suite à l'éclairement de ladite zone correspondante ZC, ZS est mesuré pour ladite valeur donnée de la longueur d'onde $\lambda$ du faisceau lumineux et à l'aide du spectromètre 16 lors de l'étape suivante 110.

Les étapes 100 et 110 sont réitérées automatiquement par le système de calcul 10 en faisant varier la longueur d'onde $\lambda$ du faisceau lumineux dans un intervalle prédéterminé. L'intervalle prédéterminé des valeurs de la longueur d'onde $\lambda$ du faisceau lumineux est, par exemple, l'intervalle compris entre 400 nm et 1000 nm, de préférence l'intervalle compris entre 450 nm et 900 nm, de préférence encore l'intervalle compris entre 450 nm et 800 nm. Le pas entre deux valeurs successives de la longueur d'onde $\lambda$, également appelé pas d'échantillonnage, est, par exemple, égal à 1 nm, de préférence égal à 0,5 nm, et de préférence encore égal à 0,33 nm.

D'une façon générale, le nombre minimal de valeurs de la longueur d'onde $\lambda$ à considérer correspond au nombre de chromophores considérés. On comprendra que plus le nombre de valeurs de la longueur d'onde $\lambda$ est élevé, plus l'indicateur final est précis.

La durée de la phase correspondant à l'ensemble des étapes 100 et 110 réitérées pour la pluralité de valeurs de la longueur d'onde $\lambda$, également appelée phase d'acquisition, est inférieure à une dizaine de secondes pour la zone correspondante ZC, ZS. Cette durée est susceptible d'être encore plus courte en fonction de l'intervalle prédéterminé des valeurs de la longueur d'onde $\lambda$ et de la valeur choisie du pas d'échantillonnage, étant entendu que plus la valeur du pas d'échantillonnage sera élevée, plus la durée de la phase d'acquisition sera courte.

Le spectre mesuré M, c'est-à-dire le spectre du signal rétrodiffusé, vérifie l'équation générale :

$$M = S \times R \times G \times D \tag{1}$$

où S représente l'intensité de la source de lumière 14,
R représente la réflectance correspondant à la diffusion de la lumière dans les tissus de la peau 12,
G représente l'efficacité de collection au niveau de la sonde 22 en contact avec la peau 12, avec 0 < G < 1, et
D représente la réponse du spectromètre 16.

Lorsque les valeurs de l'intensité S de la source, de l'efficacité de collection G et de la réponse D du spectromètre sont connues, alors la valeur de la réflectance R est obtenue directement à l'aide du spectre mesuré M et pour chaque valeur de la longueur d'onde $\lambda$ d'après l'équation (1).

Selon une première variante, lorsque les valeurs de l'intensité S de la source et de la réponse D du spectromètre ne sont pas connues, une mesure de calibration est effectuée préalablement aux étapes 100 et 110 sur un échantillon de référence, également appelé fantôme.

Le spectre mesuré pour le fantôme est alors noté $M_{std}$ et vérifie l'équation suivante :

$$M_{std} = S \times R_{std} \times G_{std} \times D \tag{2}$$

Le spectre mesuré pour la peau 12 est alors noté $M_{skin}$ et vérifie l'équation suivante :

$$M_{skin} = S \times R_{skin} \times G_{skin} \times D \tag{3}$$

Un premier ratio B du spectre mesuré pour la peau 12 sur le spectre mesuré pour le fantôme est alors calculé, et permet de déterminer la réflectance $R_{skin}$ de la peau 12 en s'affranchissant des valeurs de l'intensité S de la source et de la réponse R du spectromètre, d'après les équations suivantes :

$$B = \frac{M_{skin}}{M_{std}} = \frac{S \times R_{skin} \times G_{skin} \times D}{S \times R_{std} \times G_{std} \times D} = \frac{R_{skin} \times G_{skin}}{R_{std} \times G_{std}} \tag{4}$$

$$R_{skin} = B \times R_{std} \times \frac{G_{std}}{G_{skin}} \tag{5}$$

puis

$$R_{skin} = B \times R_{std} \tag{6}$$

en considérant en outre que l'efficacité de collection $G_{skin}$ lorsque le spectre est mesuré pour la peau 12 est sensiblement égale à l'efficacité de collection $G_{std}$ lorsque le spectre est mesuré pour le fantôme.

Selon une deuxième variante, lorsque les valeurs de l'intensité S de la source et de la réponse R du spectromètre ne sont pas connues, une mesure de prétraitement est effectuée à l'aide du retour d'excitation 27, et vérifie l'équation suivante :

$$M_{directe} = S \times G_{directe} \times D \times k \tag{7}$$

où k représente le coefficient d'atténuation de l'intensité dans la fibre optique correspondant au retour d'excitation 27 par rapport à celle dans les fibres de détection 26.

Un deuxième ratio C du spectre mesuré pour la peau 12 sur le spectre mesuré directement via le retour d'excitation 27 est alors calculé, et permet de déterminer la réflectance $R_{skin}$ de la peau 12 en s'affranchissant des valeurs de l'intensité S de la source et de la réponse R du spectromètre, d'après les équations suivantes :

$$C = \frac{M_{skin}}{M_{direct}} = \frac{S \times R_{skin} \times G_{skin} \times D}{S \times G_{direct} \times k} = \frac{R_{skin} \times G_{skin}}{G_{std} \times k} \tag{8}$$

$$R_{skin} = C \times k \times \frac{G_{direct}}{G_{skin}} \tag{9}$$

puis

$$R_{skin} = C \times k \tag{10}$$

en considérant en outre que l'efficacité de collection $G_{skin}$ lorsque le spectre est mesuré pour la peau 12 est sensiblement égale à l'efficacité de collection $G_{direct}$ lorsque le spectre est mesuré directement via le retour d'excitation 27.

Ainsi, d'une façon générale, à partir d'une mesure M(λ) de la lumière rétrodiffusée, à une longueur d'onde donnée λ, on détermine la réflectance R(λ) du tissu examiné à cette même longueur d'onde λ. On s'affranchit alors de l'influence de la source de lumière 14 et du spectromètre 16 utilisés. La réflectance correspond à la réponse du tissu à une excitation lumineuse, en fonction de ses propriétés de d'absorption et de diffusion.

En complément, lorsque que le système de calcul 10 comprend une pluralité de fibres optiques de détection 26 disposées à différentes distances $D_i$ prédéterminées de la fibre optique d'excitation 24, la réflectance R est calculée pour la pluralité de valeurs de la longueur d'onde λ et pour chacune desdites distances $D_i$ prédéterminées, comme représenté sur la figure 4.

Sur la figure 4, les courbes 250, 260, 265, 270, 275 et 280 correspondent alors aux réflectances R obtenues pour six distances prédéterminées $D_i$ distinctes, à partir de mesures M, la relation entre mesure et réflectance étant obtenue comme précédemment décrit. La courbe 250 correspond à la réflectance R à la plus petite distance $D_1$ parmi lesdites distances $D_i$, et la courbe 280 correspond à la réflectance R calculée à la plus grande distance $D_N$ parmi lesdites distances $D_i$. La courbe 255 correspond à la réflectance R obtenue pour le retour d'excitation 27. On observe alors que plus la distance $D_i$ entre la fibre d'excitation 24 et la fibre de détection 26 correspondante est élevée, plus l'amplitude de la réflectance R est faible. Ceci est dû au fait que les photons auront voyagé plus profondément dans la peau 12, et seront moins nombreux à ressortir à la distance $D_N$ de la fibre d'excitation 24 compte tenu de l'absorption des photons par la peau 12.

Les inventeurs ont constaté que des résultats exploitables sont obtenus en mettant en oeuvre des fibres de détection disposées selon 4 distances, sous réserve que la distance maximale $D_N$ soit au moins de 2 mm.

A l'issue de la phase d'acquisition correspondant à l'ensemble des étapes 100 et 110 réitérées pour la pluralité de valeurs de la longueur d'onde λ, une valeur du coefficient d'absorption $\mu_a$ et une valeur du coefficient de diffusion $\mu_s$ de la zone correspondante ZC, ZS sont déterminées, lors de l'étape 120 à l'aide du premier logiciel de détermination 34 pour au moins une valeur de la longueur d'onde λ du faisceau lumineux d'excitation et à partir du spectre mesuré M. En particulier, les valeurs du coefficient d'absorption $\mu_a$ et du coefficient de diffusion $\mu_s$ de la zone correspondante ZC, ZS sont déterminées à l'aide de la réflectance R déterminée précédemment.

Dans l'exemple de réalisation décrit, les valeurs du coefficient d'absorption $\mu_a$ et du coefficient de diffusion $\mu_s$ de la zone correspondante ZC, ZS sont déterminées pour chacune des valeurs de la longueur d'onde λ précédemment utilisées lors des étapes 100 et 110, et les valeurs du coefficient d'absorption et du coefficient de diffusion sont alors respectivement notées $\mu_a(\lambda)$ et $\mu_s(\lambda)$.

En complément, lorsque que le système de calcul 10 comprend une pluralité de fibres optiques de détection 26 disposées à différentes distances $D_i$ prédéterminées de la fibre optique d'excitation 24, les valeurs du coefficient d'absorption $\mu_a(\lambda)$ et du coefficient de diffusion $\mu_s(\lambda)$ sont déterminées en fonction des spectres M mesurés pour chacune desdites distances $D_i$ prédéterminées. En particulier, les valeurs du coefficient d'absorption $\mu_a(\lambda)$ et du coefficient de diffusion $\mu_s(\lambda)$ de la zone correspondante ZC, ZS sont déterminées à l'aide de la réflectance R déterminée à partir de chaque spectre mesuré pour chacune desdites distances $D_i$ prédéterminées.

En complément encore, une table de référence LUT (de l'anglais LookUp Table) est prédéterminée avant l'étape de détermination 120, par exemple préalablement aux étapes 100 et 110. La table de référence LUT comporte une pluralité de valeurs de la réflectance de la peau 12, chaque valeur de ladite table LUT étant prédéterminée pour un couple respectif de valeurs du coefficient d'absorption $\mu_a$ et du coefficient de diffusion $\mu_s$. La prédétermination de la table de référence LUT est effectuée, par exemple, à l'aide d'une simulation numérique de type Monte-Carlo.

De façon alternative, la table de référence LUT est déterminée analytiquement, par exemple selon la méthode décrite dans l'article de Farrell et al, intitulé « A diffusion theory model of spatially resolved, steady-state diffuse réflectance for the non-invasive détermination of tissue optical properties in vivo » et publié dans Medical Physics en 1992. La réflectance R est alors décrite comme une fonction des coefficients d'absorption $\mu_a$ et de diffusion réduit $\mu_{s'}$ de la peau 12, de la distance $D_i$ entre fibres d'excitation 24 et de détection 26, également notée r, et de l'indice de réfraction du milieu n, selon l'équation suivante :

$$R = f(\mu_a, \mu_{s'}, r, n) \tag{11}$$

L'indice de réfraction n est, par exemple, considéré égal à 1,36 ce qui correspond à un indice moyen pour les tissus biologiques

Un paramètre A relatif aux réflexions aux interfaces et dépendant des indices de réfraction du milieu ambiant $n_{ambiant}$ et des tissus $n_{tissus}$ permet alors de traiter des conditions de bord selon les équations suivantes :

$$A = \frac{1 + r_i}{1 - r_i} \tag{12}$$

$$r_i = -1{,}440 \cdot n_r^{-2} + 0{,}710 \cdot n_r^{-1} + 0{,}0636 \cdot n_r + 0{,}668 \qquad (13)$$

$$n_r = \frac{n_{tissus}}{n_{ambiant}} \qquad (14)$$

L'homme du métier notera que si le milieu ambiant est l'air, alors $n_r$ est égal à $n_{tissus}$.

La réflectance est alors calculée comme étant la densité de courant donnée par l'équation de la diffusion, perpendiculaire à la surface de sortie en r égal à 0.

En posant :

$$z_0 = \frac{1}{\mu_a + \mu_s'}$$
$$\mu_{eff} = \sqrt{3\mu_a(\mu_a + \mu_s')} \qquad (15)$$

on obtient alors :

$$R = \frac{z_0}{4\pi} \times \left[ \left( \mu_{eff} + \frac{1}{r_1} \right) \frac{e^{-\mu_{eff} r_1}}{r_1^2} + \left( 1 + \frac{4}{3}A \right) \left( \mu_{eff} + \frac{1}{r_2} \right) \frac{e^{-\mu_{eff} r_2}}{r_2^2} \right]$$

avec :

$$(16)$$

$$r_1 = \sqrt{z_0^2 + r^2}$$
$$r_2 = \sqrt{z_0^2 \left( 1 + \frac{4}{3}A \right)^2 + r^2}$$

Par convention, la réflectance ainsi prédéterminée est alors notée $R_{LUT}$, la réflectance mesurée lors des étapes 100 et 110 étant alors notée $R_{mesure}$.

En complément, les mesures sont ensuite traitées afin de les rendre comparables aux valeurs de la table de référence LUT. La courbe de réflectance à comparer à la base de données est calculée comme suit. Pour une longueur d'onde $\lambda$ donnée : $R_{ref\text{-}LUT}$ correspond à la courbe de réflectance calculée par le programme Monte Carlo pour les paramètres optiques du fantôme de référence, ces paramètres étant toujours connus. $R_{ref\text{-}mesure}$ correspond à la mesure réalisée sur ce fantôme. Enfin, $R_{mesure}$ est la réflectance mesurée pour la zone correspondante ZC, ZS. Une réflectance recalée, notée $R_{inconnu\text{-}LUT}$, est alors obtenue à l'aide de l'équation suivante :

$$R_{inconnu\text{-}LUT} = R_{mesure} \times \frac{R_{ref\text{-}LUT}}{R_{ref\text{-}mesure}} \qquad (17)$$

De manière analogue, lorsque que le système de calcul 10 comprend une pluralité de fibres optiques de détection 26 disposées à différentes distances $D_i$ prédéterminées de la fibre optique d'excitation 24, la prédétermination de la table de référence LUT est effectuée pour chacune desdites distances $D_i$ prédéterminées, chaque valeur de ladite table LUT étant prédéterminée pour un couple respectif de valeurs du coefficient d'absorption et du coefficient de diffusion et pour lesdites différentes distances $D_i$, comme représenté sur la figure 4. Par convention, la réflectance ainsi prédéterminée est alors notée $R_{LUT}^{Di}$ pour chaque distance Di respective, la réflectance mesurée lors des étapes 100 et 110 étant alors notée $R_{mesure}^{Di}$ pour chaque distance Di respective.

Sur la figure 4, les nappes 200, 205, 210, 215, 220 et 225 correspondent alors aux réflectances $R_{LUT,Di}$ prédéterminées pour les six distances prédéterminées $D_i$ distinctes, la nappe 200 correspondant à la réflectance $R_{LUT,D1}$ prédéterminée pour la plus petite distance $D_1$ parmi lesdites distances $D_i$, et la nappe 225 correspondant à la réflectance

$R_{LUT,DN}$ prédéterminée pour la plus grande distance $D_N$ parmi lesdites distances $D_i$. On observe alors également que plus la distance entre la fibre d'excitation 24 et la fibre de détection 26 correspondante est élevée, plus l'amplitude de la réflectance R est faible. La nappe 230 correspond à la réflectance R obtenue pour le retour d'excitation 27.

Lors de l'étape de détermination 120, le couple de valeurs déterminées du coefficient d'absorption $\mu_a$ et du coefficient de diffusion $\mu_s$ est alors celui minimisant l'erreur entre les réflectances prédéterminées $R_{LUT}$ de la table de référence LUT et la ou les valeurs de réflectance $R_{mesure}$ mesurées pour une valeur donnée de la longueur d'onde $\lambda$.

Le couple de valeurs déterminées du coefficient d'absorption $\mu_a$ et du coefficient de diffusion $\mu_s$ vérifie, par exemple, l'équation suivante :

$$(\mu_a,\mu_s) = Arg \min_{(\mu_a,\mu_s)} \sqrt{\sum_{i=1}^{N}(R_{LUT}^{D_i} - R_{mesure}^{D_i})^2} \qquad (18)$$

La figure 4 illustre à titre d'exemple la détermination du couple $(\mu_a, \mu_s)$ obtenu pour la valeur d'onde $\lambda_0$, celui-ci étant alors noté $(\mu_a(\lambda_0), \mu_s(\lambda_0))$.

Cette étape de détermination 120 est réitérée pour chacune des valeurs de la longueur d'onde $\lambda$ dans l'intervalle et selon le pas d'échantillonnage prédéterminés, suivant les valeurs décrites précédemment.

La concentration d'au moins un chromophore de la peau est ensuite calculée, lors de l'étape 130, à partir de la ou chaque valeur déterminée du coefficient d'absorption $\mu_a(\lambda)$ et à l'aide du premier logiciel de calcul 38.

Le coefficient d'absorption $\mu_a(\lambda)$ vérifie l'équation suivante :

$$\mu_a(\lambda) = Deox\mu_{ahb}(\lambda) + Ox\mu_{ahbO2}(\lambda) + W\mu_{aeau}(\lambda) + Bil\mu_{aBil}(\lambda) + Fat\mu_{aFat}(\lambda) + L_{epi}/L_{derm}*Mel\mu_{aMel}(\lambda)$$

$$(19)$$

où Deox représente la concentration de déoxyhémoglobine dans la zone correspondante ZC, ZS de la peau 12,

Ox représente la concentration d'oxyhémoglobine dans ladite zone de la peau 12,
W représente la concentration d'eau dans ladite zone de la peau 12,
Bil représente la concentration de bilirubine dans ladite zone de la peau 12,
Fat représente la concentration de graisse dans ladite zone de la peau 12, et
Mel représente la fraction de mélanosomes présents dans un épiderme d'épaisseur $L_{epi}$ supposée égale à 60 $\mu$m pour une épaisseur totale de derme $L_{derm}$ supposée égale à 1,5 mm.

Précisons que les termes $\mu_{ahb}(\lambda)$, $\mu_{ahbO2}(\lambda)$, $\mu_{aeau}(\lambda)$, $\mu_{aBil}(\lambda)$, $\mu_{aFat}(\lambda)$ et $\mu_{aMel}(\lambda)$ représentent respectivement les coefficients d'absorption de la déoxyhémoglobine, de l'oxyhémoglobine, de l'eau, de la bilirubine et de la graisse pour une concentration unitaire.

Le ou chaque chromophore est choisi parmi le groupe constitué de : l'eau, la mélanine, l'oxyhémoglobine, la déoxyhémoglobine et la bilirubine, la concentration en oxyhémoglobine $HbO_2$ et la concentration en déoxyhémoglobine Hb étant calculées de préférence.

Les concentrations de bilirubine Bil et de graisse Fat ne sont, par exemple, pas pris en compte, les grandeurs Bil.$\mu_{aBil}$ et Fat.$\mu_{aFat}$ étant supposées négligeables pour les valeurs de la longueur d'onde $\lambda$ comprises entre 400 nm et 1000 nm. Autrement dit, on considère que la contribution de la bilirubine et de la graisse à l'absorption est négligeable devant les autres chromophores pour cette plage de valeurs de la longueur d'onde $\lambda$, pour la réaction dermique étudiée.

En complément, lors de l'étape 130 de calcul de la concentration du ou des chromophores $HbO_2$, Hb, la concentration du ou des chromophores $HbO_2$, Hb est d'abord calculée pour la zone saine ZS de la peau 12, visible sur la figure 1, à partir de la ou des valeurs du coefficient d'absorption $\mu_a$ de ladite zone saine ZS, et la concentration du ou des chromophores $HbO_2$, Hb est ensuite calculée pour la zone à caractériser ZC à partir de la concentration du ou des chromophores pour la zone saine ZS et de la ou des valeurs du coefficient d'absorption $\mu_a$ de ladite zone à caractériser ZC.

Sur la figure 5, la courbe 300 représente les valeurs prises par le coefficient de diffusion $\mu_s$ de la zone saine ZS pour les valeurs de la longueur d'onde $\lambda$ comprises entre 400 nm et 900 nm, et la courbe 310 représente les valeurs prises par le coefficient de diffusion $\mu_s$ de la zone à caractériser ZC pour le même intervalle de valeurs de la longueur d'onde $\lambda$.

Sur la figure 6, la courbe 350 représente les valeurs prises par le coefficient de d'absorption $\mu_a$ de la zone saine ZS pour les valeurs de la longueur d'onde $\lambda$ comprises entre 400 nm et 900 nm, et la courbe 360 représente les valeurs prises par le coefficient d'absorption $\mu_a$ de la zone à caractériser ZC pour le même intervalle de valeurs de la longueur d'onde $\lambda$.

Selon ce complément, les étapes 100, 110 et 120 décrites précédemment sont alors effectuées dans un premier temps pour la zone saine ZS, puis dans un second temps pour la zone à caractériser ZC.

La concentration en oxyhémoglobine $HbO_2$, la concentration en déoxyhémoglobine Hb, la concentration en mélanine Mel et la concentration d'eau W sont d'abord calculés, à l'aide du premier logiciel de calcul 38, pour la zone saine ZS à partir des valeurs du coefficient d'absorption $\mu_a$ de ladite zone saine ZS et de valeurs initiales standards des concentrations en oxyhémoglobine $HbO_2$, en déoxyhémoglobine Hb, en mélanine Mel et la concentration d'eau W. Ces valeurs standards sont par exemple issues de la littérature.

En supposant que la grandeur $Mel.\mu_{aMel}$ ne varie pas entre la zone saine ZS et la zone à caractériser ZC, la concentration en oxyhémoglobine $HbO_2$ et la concentration en déoxyhémoglobine Hb sont ensuite calculées, à l'aide du premier logiciel de calcul 38, pour la zone à caractériser ZC à partir des valeurs du coefficient d'absorption $\mu_a$ de ladite zone à caractériser ZC et de valeurs initiales des concentrations égales aux dernières valeurs calculées précédemment pour la zone saine ZS. L'initialisation de l'algorithme à partir de valeurs issues de la zone saine ZS permet une meilleure prise en compte du phototype de l'individu.

L'indicateur IND de quantification de la réaction cutanée est enfin calculé, lors de l'étape 140, à l'aide du deuxième logiciel de calcul 40 et en fonction de la concentration du ou de chaque chromophore $HbO_2$, Hb précédemment calculée et en fonction également de la ou chaque valeur déterminée du coefficient de diffusion $\mu_s$ de la zone à caractériser ZC.

L'indicateur de quantification IND est de préférence calculé en fonction d'une moyenne, telle que la moyenne arithmétique, d'une pluralité de valeurs du coefficient de diffusion $\mu_s$ de la zone à caractériser ZC, ces valeurs du coefficient de diffusion $\mu_s$ étant déterminées pour une pluralité de valeurs de la longueur d'onde $\lambda$ par exemple comprises entre 450 nm et 800 nm, de préférence comprises entre 650 nm et 800 nm, de préférence encore comprises entre 740 nm et 760 nm.

L'indicateur de quantification IND vérifie alors, par exemple, l'équation suivante, pas couverte par le texte des revendications, mais considérée comme utile à la compréhension de l'invention :

$$IND = \alpha_1 + \beta_1 \times Ox + \gamma_1 \times Deox + \delta_1 \times Dif \qquad (20)$$

où IND représente l'indicateur de quantification de la réaction dermique,

$\alpha_1$, $\beta_1$, $\gamma_1$ et $\delta_1$ sont des coefficients prédéterminés pour une valeur donnée du délai entre l'instant de mesure du spectre M du rayonnement rétrodiffusé et l'instant d'injection du principe actif, avec $\beta_1$, $\gamma_1$ et $\delta_1$ de valeurs non nulles, Dif représente la moyenne, telle que la moyenne arithmétique, de la ou des valeurs déterminées du coefficient de diffusion $\mu_s$ calculée pour les intervalles de longueur d'onde indiqués ci-dessus, à savoir [450 nm ; 800 nm], de préférence [650 nm ; 800 nm], et de préférence encore [740 nm ; 760 nm].

Les valeurs des coefficients $\alpha_1$, $\beta_1$, $\gamma_1$ et $\delta_1$ sont, par exemple, prédéterminées de manière empirique à partir de tests réalisés sur un ensemble de patients. Cette prédétermination est faite de préférence via une analyse factorielle discriminante. En variante, la prédétermination est faite via une analyse en composante principale.

En variante, l'indicateur de quantification IND est calculé en fonction d'un écart entre les concentrations du ou de chaque chromophore Hb, $HbO_2$ de la zone saine ZS et de la zone à caractériser ZC et en fonction du ou des écarts entre les valeurs déterminées du coefficient de diffusion $\mu_s$ de la zone saine ZS et de la zone à caractériser ZC.

Selon cette variante, l'indicateur de quantification IND vérifie alors, par exemple, l'équation suivante, pas couverte par le texte des revendications, mais considérée comme utile à la compréhension de l'invention :

$$IND = \alpha'_1 + \beta'_1 \times \Delta Ox + \gamma'_1 \times \Delta Deox + \delta'_1 \times \Delta Dif \qquad (21)$$

où IND représente l'indicateur de quantification de la réaction dermique,
$\alpha'_1$, $\beta'_1$, $\gamma'_1$ et $\delta'_1$ sont des coefficients prédéterminés pour une valeur donnée du délai entre l'instant de mesure du spectre M du rayonnement rétrodiffusé et l'instant d'injection du principe actif, avec $\beta'_1$, $\gamma'_1$ et $\delta'_1$ de valeurs non nulles $\Delta Ox$ et $\Delta Deox$ représentent un écart entre les concentrations en oxyhémoglobine $HbO_2$ de la zone saine ZS et de la zone à caractériser ZC, et respectivement un écart entre les concentrations en déoxyhémoglobine Hb de la zone saine ZS et de la zone à caractériser ZC, et
$\Delta Dif$ représente une moyenne, telle que la moyenne arithmétique, du ou des écarts entre les valeurs déterminées du coefficient de diffusion $\mu_s$ de la zone saine ZS et de la zone à caractériser ZC, la moyenne étant par exemple calculée pour les intervalles de longueur d'onde indiqués ci-dessus, à savoir [450 nm ; 800 nm], de préférence [650 nm ; 800 nm], et de préférence encore [740 nm ; 760 nm].

De manière analogue, les valeurs des coefficients $\alpha'_1$, $\beta'_1$, $\gamma'_1$ et $\delta'_1$ sont, par exemple, prédéterminées de manière empirique à partir de tests réalisés sur un ensemble de patients.

A titre d'exemple, pour un test à la tuberculine, les valeurs des coefficients $\alpha'_1$, $\beta'_1$, $\gamma'_1$ et $\delta'_1$ sont respectivement égales à 1,741, -0,007, -0,147 et -0,434, comme cela sera décrit par la suite pour l'équation (23).

Les résultats obtenus avec le procédé de l'état de la technique pour un groupe de 48 patients lors d'essais cliniques sont illustrés sur l'histogramme de la figure 7 représentant, sous forme de barres 400, les valeurs de l'indicateur de quantification IND calculées pour chacun des 48 tests sur patients, les tests étant identifiés par des numéros respectifs variant en abscisse de 1 à 48. Dans l'exemple de la figure 7 correspondant au procédé de l'état de la technique, l'indicateur de quantification IND vérifie l'équation suivante :

$$IND = -0{,}070 - 0{,}128 \times Ox - 0{,}026 \times Deox \qquad (22)$$

On constate alors la présence de nombreuses erreurs de diagnostic pour le test de la réaction dermique, à savoir des cas où la réaction est indiquée à tort comme étant positive (faux positif), les barres étant alors référencées 400A, et au contraire des cas où la réaction est indiquée négative alors qu'elle est en réalité positive (faux négatif), les barres étant alors référencées 400B. Dans l'exemple de la figure 7, 17 cas de faux positif et 3 cas de faux négatif sont dénombrés, soit 20 erreurs pour 48 tests, c'est-à-dire un taux d'erreur égal à 42%.

En comparaison, les résultats obtenus avec le procédé selon l'invention pour le même groupe de 48 tests sur patients sont illustrés sur l'histogramme de la figure 8 représentant sous forme de barres 500 les valeurs de l'indicateur de quantification IND calculées pour chacun desdits tests, également identifiés par des numéros respectifs variant en abscisse de 1 à 48. Les résultats de la figure 8 correspondent au cas où l'indicateur de quantification IND est calculé d'après l'équation (21). Dans l'exemple de la figure 8, l'indicateur de quantification IND vérifie l'équation suivante, pas couverte par le texte des revendications, mais considérée comme utile à la compréhension de l'invention :

$$IND = 1{,}741 - 0{,}007 \times \Delta Ox - 0{,}147 \times \Delta Deox - 0{,}434 \times \Delta Dif \qquad (23)$$

On constate alors que le nombre d'erreurs de diagnostic est bien moindre avec le procédé selon l'invention qu'avec le procédé de l'état de la technique. En effet, dans l'exemple de la figure 8, seulement 5 erreurs sont dénombrées pour 48 tests, soit un taux d'erreur égal à 10%, c'est-à-dire sensiblement 4 fois moins que le taux d'erreur de l'état de la technique. Les erreurs sont dans cet exemple 4 cas de faux positif, les barres étant alors référencées 500A, et 1 cas de faux négatif, ladite barre étant référencée 500B.

Dans l'exemple des figures 7 et 8, les indicateurs de quantification IND sont calculés 72 heures après la stimulation à l'origine de la réaction dermique.

Il a alors été constaté qu'il est possible de calculer dans un délai inférieur à 72 heures l'indicateur de quantification IND suivant le procédé de calcul selon l'invention, tout en conservant un taux d'erreur faible. Ainsi, des indicateurs suffisamment fiables ont pu être établis, permettant une quantification plus précoce de la réaction, le délai entre l'injection et la lecture pouvant être réduit à 18 heures.

Avec le procédé selon l'invention, la prise en compte du coefficient de diffusion $\mu_s$ dans le calcul de l'indicateur de quantification IND permet notamment de mieux détecter une induration.

Il a en outre été constaté que les résultats obtenus sont meilleurs lorsque l'indicateur de quantification IND est calculé d'après l'équation (21), plutôt que d'après l'équation (20).

On conçoit alors que le procédé et le système de calcul 10 selon l'invention permettent d'améliorer la pertinence de l'indicateur de quantification IND calculé, tout en permettant de calculer cet indicateur IND à plus bref délai après la stimulation à l'origine de la réaction dermique.

La figure 9 correspond à un deuxième mode de réalisation pour lequel les éléments identiques au premier mode de réalisation décrit précédemment sont repérés par des références identiques, et ne sont pas décrits à nouveau.

Selon ce deuxième mode de réalisation, le deuxième logiciel de calcul 40 est propre à calculer l'indicateur de quantification IND en fonction de la concentration du ou de chaque chromophore précédemment calculée, de la ou chaque valeur déterminée du coefficient de diffusion $\mu_s$, et en fonction également de la ou chaque valeur déterminée du coefficient d'absorption $\mu_a$.

En variante, le deuxième logiciel de calcul 40 est propre à calculer l'indicateur de quantification IND en fonction d'un écart entre les concentrations du ou de chaque chromophore Hb, $HbO_2$ de la zone saine ZS et de la zone à caractériser ZC, en fonction du ou des écarts entre les valeurs déterminées du coefficient de diffusion $\mu_s$ de la zone

saine ZS et de la zone à caractériser ZC et en fonction du ou des écarts entre les valeurs déterminées du coefficient d'absorption $\mu_a$ de la zone saine ZS et de la zone à caractériser ZC.

Selon ce deuxième mode de réalisation, l'indicateur de IND, calculé lors de l'étape 140 à l'aide du deuxième logiciel de calcul 40, est fonction de la concentration du ou de chaque chromophore $HbO_2$, Hb précédemment calculée, de la ou chaque valeur déterminée du coefficient de diffusion $\mu_s$ de la zone à caractériser ZC et également de la ou chaque valeur déterminée du coefficient d'absorption $\mu_a$ de la zone à caractériser ZC.

L'indicateur de quantification IND est de préférence calculé en fonction d'une moyenne, telle que la moyenne arithmétique, d'une pluralité de valeurs du coefficient de diffusion $\mu_s$ de la zone à caractériser ZC, ces valeurs du coefficient de diffusion $\mu_s$ étant déterminées pour une pluralité de valeurs de la longueur d'onde $\lambda$ par exemple comprises entre 450 nm et 800 nm, de préférence comprises entre 650 nm et 800 nm, de préférence encore comprises entre 740 nm et 760 nm.

L'indicateur de quantification IND est également de préférence calculé en fonction d'une moyenne, telle que la moyenne arithmétique, d'une pluralité de valeurs du coefficient d'absorption $\mu_a$ de la zone à caractériser ZC, ces valeurs du coefficient d'absorption $\mu_a$ étant déterminées pour une pluralité de valeurs de la longueur d'onde du faisceau lumineux par exemple comprises entre 450 nm et 800 nm, de préférence comprises entre 450 nm et 700 nm, de préférence encore comprises entre 500 nm et 650 nm.

L'indicateur de quantification IND vérifie alors, par exemple, l'équation suivante :

$$\text{IND} = \alpha_2 + \beta_2 \times \text{Ox} + \gamma_2 \times \text{Deox} + \delta_2 \times \text{Dif} + \varepsilon_2 \times \text{Abs} \qquad (24)$$

où IND représente l'indicateur de quantification de la réaction dermique,

$\alpha_2$, $\beta_2$, $\gamma_2$, $\delta_2$ et $\varepsilon_2$ sont des coefficients prédéterminés pour une valeur donnée du délai entre l'instant de mesure du spectre M du rayonnement rétrodiffusé et l'instant d'injection du principe actif, avec $\beta_2$, $\gamma_2$, $\delta_2$ et $\varepsilon_2$ de valeurs non nulles,

Dif représente la moyenne, telle que la moyenne arithmétique, de la ou des valeurs déterminées du coefficient de diffusion $\mu_s$ calculée pour les intervalles de longueur d'onde indiqués ci-dessus, à savoir [450 nm ; 800 nm], de préférence [650 nm ; 800 nm], et de préférence encore [740 nm ; 760 nm], et

Abs représente la moyenne, telle que la moyenne arithmétique, de la ou des valeurs déterminées du coefficient d'absorption $\mu_a$ calculée pour les intervalles de longueur d'onde indiqués ci-dessus, à savoir [450 nm ; 800 nm], de préférence [450 nm ; 700 nm], et de préférence encore [500 nm ; 650 nm].

Les valeurs des coefficients $\alpha_2$, $\beta_2$, $\gamma_2$, $\delta_2$ et $\varepsilon_2$ sont, par exemple, prédéterminées de manière empirique à partir de tests réalisés sur un ensemble de patients. Cette prédétermination est faite de préférence via une analyse factorielle discriminante. En variante, la prédétermination est faite de préférence via une analyse en composante principale.

En variante, l'indicateur de quantification IND est calculé en fonction d'un écart entre les concentrations du ou de chaque chromophore Hb, $HbO_2$ de la zone saine ZS et de la zone à caractériser ZC, en fonction du ou des écarts entre les valeurs déterminées du coefficient de diffusion $\mu_s$ de la zone saine ZS et de la zone à caractériser ZC et en fonction du ou des écarts entre les valeurs déterminées du coefficient d'absorption $\mu_a$ de la zone saine ZS et de la zone à caractériser ZC.

Selon cette variante, l'indicateur de quantification IND vérifie alors, par exemple, l'équation suivante :

$$\text{IND} = \alpha'_2 + \beta'_2 \times \Delta\text{Ox} + \gamma'_2 \times \Delta\text{Deox} + \delta'_2 \times \Delta\text{Dif} + \varepsilon'_2 \times \Delta\text{Abs} \qquad (25)$$

où IND représente l'indicateur de quantification de la réaction dermique,

$\alpha'_2$, $\beta'_2$, $\gamma'_2$, $\delta'_2$ et $\varepsilon'_2$ sont des coefficients prédéterminés pour une valeur donnée du délai entre l'instant de mesure du spectre d'un rayonnement rétrodiffusé et l'instant d'injection du principe actif, avec $\beta'_2$, $\gamma'_2$ et $\delta'_2$ de valeurs non nulles,

$\Delta\text{Ox}$ et $\Delta\text{Deox}$ représentent un écart entre les concentrations en oxyhémoglobine $HbO_2$ de la zone saine ZS et de la zone à caractériser ZC, et respectivement un écart entre les concentrations en déoxyhémoglobine Hb de la zone saine ZS et de la zone à caractériser ZC, et

$\Delta\text{Dif}$ et $\Delta\text{Abs}$ représentent une moyenne, telle que la moyenne arithmétique, du ou des écarts entre les valeurs déterminées du coefficient de diffusion $\mu_s$ de la zone saine ZS et de la zone à caractériser ZC, et respectivement du ou des écarts entre les valeurs déterminées du coefficient d'absorption $\mu_a$ de la zone saine ZS et de la zone à caractériser ZC.

La moyenne pour obtenir ΔDif est, par exemple, calculée pour les intervalles de longueur d'onde indiqués ci-dessus, à savoir [450 nm ; 800 nm], de préférence [650 nm ; 800 nm], et de préférence encore [740 nm ; 760 nm], et celle pour obtenir ΔAbs est, par exemple, calculée pour les intervalles de longueur d'onde précités, à savoir [450 nm ; 800 nm], de préférence [450 nm ; 700 nm], et de préférence encore [500 nm ; 650 nm].

De manière analogue, les valeurs des coefficients $\alpha'_2$, $\beta'_2$, $\gamma'_2$, $\delta'_2$ et $\varepsilon'_2$ sont, par exemple, prédéterminées de manière empirique à partir de tests réalisés sur un ensemble de patients.

A titre d'exemple, les valeurs des coefficients $\alpha_2$, $\beta_2$, $\gamma_2$, $\delta_2$ et $\varepsilon_2$ sont respectivement égales à 1,841, -0,128, -0,181, -0,463 et 0,324, comme cela sera décrit par la suite pour l'équation (26) ci-dessous.

Les résultats obtenus avec le procédé selon le deuxième mode de réalisation de l'invention pour le même groupe de 48 tests, que celui indiqué pour les figures 7 et 8 précédentes, sont illustrés sur l'histogramme de la figure 9 représentant sous forme de barres 550 les valeurs de l'indicateur de quantification IND calculées pour chacun desdits 48 patients, également identifiés par des numéros respectifs variant en abscisse de 1 à 48. Les résultats de la figure 9 correspondent au cas où l'indicateur de quantification IND est calculé d'après l'équation (25). Dans l'exemple de la figure 9, l'indicateur de quantification IND vérifie l'équation suivante :

$$IND = 1{,}841 - 0{,}128 \times Ox - 0{,}181 \times Deox - 0{,}463 \times Dif + 0{,}324 \times Abs \qquad (26)$$

On constate alors que le nombre d'erreurs de diagnostic est encore moindre avec le procédé selon le deuxième mode de réalisation qu'avec le procédé selon le premier mode de réalisation, et *a fortiori* qu'avec le procédé de l'état de la technique. En effet, dans l'exemple de la figure 9, seulement 4 erreurs sont dénombrées pour 48 patients, soit un taux d'erreur égal à 8%, c'est-à-dire environ 5,5 fois moins que le taux d'erreur de l'état de la technique. Les erreurs sont, dans cet exemple, 3 cas de faux positif, les barres étant alors référencées 550A, et 1 cas de faux négatif, ladite barre étant référencée 550B.

Dans l'exemple des figures 7 et 9, les indicateurs de quantification IND sont calculés 72 heures après la stimulation à l'origine de la réaction dermique.

Il a alors également été constaté qu'il est possible de calculer dans un délai nettement inférieur à 72 heures l'indicateur de quantification IND suivant le procédé de calcul selon le deuxième mode de réalisation, tout en conservant un taux d'erreur faible. Ainsi, des indicateurs suffisamment fiables ont pu être établis, permettant une quantification plus précoce de la réaction, le délai entre l'injection et la lecture pouvant être réduit à 18 heures.

Il a en outre été constaté que les résultats obtenus sont meilleurs lorsque l'indicateur de quantification IND est calculé d'après l'équation (25), plutôt que d'après l'équation (24).

On conçoit alors que le procédé et le système de calcul 10 selon le deuxième mode de réalisation de l'invention permettent d'améliorer encore la pertinence de l'indicateur de quantification IND calculé, tout en permettant de calculer cet indicateur IND à plus bref délai après la stimulation à l'origine de la réaction dermique.

**Revendications**

1. Procédé de calcul d'un indicateur de quantification (IND) d'une réaction dermique de la peau (12) d'un être vivant, telle qu'une réaction dermique suite à l'injection d'un principe actif, la peau (12) comportant une pluralité de chromophores (HbO$_2$, Hb, Mel, Bil),

   le procédé comprenant les étapes suivantes :

      - l'éclairement (100) d'une zone à caractériser (ZC) de la peau (12) via un faisceau lumineux d'excitation émis par une source de lumière (14), la zone à caractériser (ZC) comportant la réaction dermique,
      - la mesure (110), à l'aide d'un spectromètre (16), du spectre (M) d'un rayonnement rétrodiffusé issu de la peau (12) suite à l'éclairement de ladite zone à caractériser (ZC),
      - la détermination (120), à partir du spectre mesuré (M) et pour au moins une valeur donnée de la longueur d'onde ($\lambda$) du faisceau lumineux, d'une valeur du coefficient d'absorption ($\mu_a$) de la zone à caractériser,
      - le calcul (130), à partir de la ou chaque valeur déterminée du coefficient d'absorption ($\mu_a$), de la concentration (Ox, Deox) d'au moins un chromophore (HbO$_2$, Hb) de la peau, et
      - le calcul (140) de l'indicateur de quantification de la réaction dermique (IND) en fonction de la concentration du ou de chaque chromophore précédemment calculée (Ox, Deox ; ΔOx, ΔDeox),

   le procédé étant **caractérisé en ce qu'**il comprend en outre la détermination (120), à partir du spectre mesuré (M) et pour au moins une valeur donnée de la longueur d'onde ($\lambda$) du faisceau lumineux, d'une valeur du

coefficient de diffusion ($\mu_s$) de la zone à caractériser,

**en ce que** l'indicateur de quantification de la réaction dermique (IND) est calculé en fonction également de la ou chaque valeur déterminée du coefficient de diffusion ($\mu_s$, Dif ; $\Delta$Dif),

**en ce que** l'indicateur de quantification de la réaction dermique (IND) vérifie l'équation suivante :

$$IND = \alpha + \beta \times Ox + \gamma \times Deox + \delta \times Dif + \varepsilon \times Abs$$

où IND représente l'indicateur de quantification de la réaction dermique,

$\alpha$, $\beta$, $\gamma$, $\delta$ et $\varepsilon$ sont des coefficients prédéterminés pour une valeur donnée du délai entre l'instant de mesure du spectre d'un rayonnement rétrodiffusé et l'instant d'injection du principe actif, avec $\beta$, $\gamma$, $\delta$ et $\varepsilon$ de valeurs non nulles,

Ox et Deox représentent la concentration en oxyhémoglobine ($HbO_2$) et respectivement en déoxyhémoglobine (Hb), pour la zone à caractériser (ZC),

Dif et Abs représentent une moyenne, telle que la moyenne arithmétique, de la ou des valeurs déterminées du coefficient de diffusion ($\mu_s$) de la zone à caractériser (ZC), et respectivement de la ou des valeurs déterminées du coefficient d'absorption ($\mu_a$) de la zone à caractériser (ZC).

2. Procédé de calcul d'un indicateur de quantification (IND) d'une réaction dermique de la peau (12) d'un être vivant, telle qu'une réaction dermique suite à l'injection d'un principe actif, la peau (12) comportant une pluralité de chromophores ($HbO_2$, Hb, Mel, Bil),

le procédé comprenant les étapes suivantes :

- l'éclairement (100) d'une zone à caractériser (ZC) de la peau (12) via un faisceau lumineux d'excitation émis par une source de lumière (14), la zone à caractériser (ZC) comportant la réaction dermique,
- la mesure (110), à l'aide d'un spectromètre (16), du spectre (M) d'un rayonnement rétrodiffusé issu de la peau (12) suite à l'éclairement de ladite zone à caractériser (ZC),
- la détermination (120), à partir du spectre mesuré (M) et pour au moins une valeur donnée de la longueur d'onde ($\lambda$) du faisceau lumineux, d'une valeur du coefficient d'absorption ($\mu_a$) de la zone à caractériser,
- le calcul (130), à partir de la ou chaque valeur déterminée du coefficient d'absorption ($\mu_a$), de la concentration (Ox, Deox) d'au moins un chromophore ($HbO_2$, Hb) de la peau, et
- le calcul (140) de l'indicateur de quantification de la réaction dermique (IND) en fonction de la concentration du ou de chaque chromophore précédemment calculée (Ox, Deox ; $\Delta$Ox, $\Delta$Deox),

le procédé étant **caractérisé en ce qu'**il comprend en outre la détermination (120), à partir du spectre mesuré (M) et pour au moins une valeur donnée de la longueur d'onde ($\lambda$) du faisceau lumineux, d'une valeur du coefficient de diffusion ($\mu_s$) de la zone à caractériser,

**en ce que** l'indicateur de quantification de la réaction dermique (IND) est calculé en fonction également de la ou chaque valeur déterminée du coefficient de diffusion ($\mu_s$, Dif ; $\Delta$Dif),

lors de l'étape (130) de calcul de la concentration du ou des chromophores ($HbO_2$, Hb), la concentration du ou des chromophores est en outre calculée pour une zone saine (ZS) de la peau (12) à partir de la ou des valeurs du coefficient d'absorption ($\mu_a$) pour ladite zone saine (ZS), et

lors de l'étape de calcul (140), l'indicateur de quantification de la réaction dermique (IND) est calculé en fonction d'un écart ($\Delta$Ox, $\Delta$Deox) entre les concentrations du ou de chaque chromophore (Hb, $HbO_2$) de la zone saine (ZS) et de la zone à caractériser (ZC) et en fonction du ou des écarts ($\Delta$Dif) entre les valeurs déterminées du coefficient de diffusion ($\mu_s$) de la zone saine (ZS) et de la zone à caractériser (ZC), et en fonction en outre du ou des écarts ($\Delta$Abs) entre les valeurs déterminées du coefficient d'absorption ($\mu_a$) pour une zone saine (ZS) de la peau (12) et de la zone à caractériser (ZC), et

l'indicateur de quantification de la réaction dermique (IND) vérifie l'équation suivante :

$$IND = \alpha' + \beta' \times \Delta Ox + \gamma' \times \Delta Deox + \delta' \times \Delta Dif + \varepsilon' \times \Delta Abs$$

où IND représente l'indicateur de quantification de la réaction dermique,

$\alpha'$, $\beta'$, $\gamma'$, $\delta'$ et $\varepsilon'$ sont des coefficients prédéterminés pour une valeur donnée du délai entre l'instant de mesure du spectre d'un rayonnement rétrodiffusé et l'instant d'injection du principe actif, avec $\beta'$, $\gamma'$, $\delta'$ et $\varepsilon'$ de valeurs non nulles,

$\Delta Ox$ et $\Delta Deox$ représentent un écart entre les concentrations en oxyhémoglobine ($HbO_2$) de la zone saine (ZS) et de la zone à caractériser (ZC), et respectivement un écart entre les concentrations en déoxyhémoglobine (Hb) de la zone saine (ZS) et de la zone à caractériser (ZC),

$\Delta Dif$ et $\Delta Abs$ représentent une moyenne, telle que la moyenne arithmétique, du ou des écarts entre les valeurs déterminées du coefficient de diffusion ($\mu_s$) de la zone saine (ZS) et de la zone à caractériser (ZC), et respectivement du ou des écarts entre les valeurs déterminées du coefficient d'absorption ($\mu_a$) de la zone saine (ZS) et de la zone à caractériser (ZC).

3. Procédé selon la revendication 1 ou 2, dans lequel, lors de l'étape de calcul (140), l'indicateur de quantification de la réaction dermique (IND) est calculé en fonction en outre de la ou chaque valeur déterminée du coefficient d'absorption ($\mu_3$, Abs ; $\Delta Abs$).

4. Procédé selon la revendication 3, dans lequel l'indicateur de quantification de la réaction dermique (IND) est calculé en fonction d'une moyenne (Abs ; $\Delta Abs$), telle que la moyenne arithmétique, d'une pluralité de valeurs du coefficient d'absorption ($\mu_a$), déterminées pour une pluralité de valeurs de la longueur d'onde ($\lambda$) du faisceau lumineux comprises entre 450 nm et 800 nm, de préférence comprises entre 450 nm et 700 nm, de préférence encore comprises entre 500 nm et 650 nm.

5. Procédé selon l'une quelconque des revendications précédentes, dans lequel l'indicateur de quantification de la réaction dermique (IND) est calculé en fonction d'une moyenne (Dif; $\Delta Dif$), telle que la moyenne arithmétique, d'une pluralité de valeurs du coefficient de diffusion ($\mu_s$), déterminées pour une pluralité de valeurs de la longueur d'onde ($\lambda$) du faisceau lumineux comprises entre 450 nm et 800 nm, de préférence comprises entre 650 nm et 800 nm, de préférence encore comprises entre 740 nm et 760 nm.

6. Procédé selon l'une quelconque des revendications précédentes, dans lequel la zone (ZS, ZC) de la peau (12) est éclairée via une fibre optique d'excitation (24), et la mesure du spectre est effectuée via une pluralité de fibres optiques de détection (26) reliées au spectromètre (16), les fibres de détection (26) étant à des distances différentes ($D_i$) de la fibre d'excitation (24), et
dans lequel la détermination, pour au moins une valeur donnée de la longueur d'onde ($\lambda$) du faisceau lumineux, de la valeur du coefficient d'absorption ($\mu_a$), respectivement du coefficient de diffusion ($\mu_s$), est effectuée en fonction des spectres (M) mesurés pour lesdites différentes distances ($D_i$).

7. Procédé selon l'une quelconque des revendications précédentes, dans lequel le procédé comprend en outre la prédétermination d'une table de référence (LUT) comportant une pluralité de valeurs ($R_{LUT}$) de la réflectance de la peau, chaque valeur ($R_{LUT}$) de ladite table étant prédéterminée pour un couple respectif de valeurs du coefficient d'absorption ($\mu_a$) et du coefficient de diffusion ($\mu_s$), dans lequel au moins une valeur ($R_{mesure}$) de la réflectance de la zone à caractériser est mesurée à l'aide du spectromètre (16) lors de l'étape de mesure (110), et dans lequel lors de l'étape (120) de détermination du coefficient d'absorption ($\mu_a$) et du coefficient de diffusion ($\mu_s$), le couple de valeurs déterminées du coefficient d'absorption ($\mu_a$) et du coefficient de diffusion ($\mu_s$) est celui minimisant l'erreur entre les réflectances prédéterminées ($R_{LUT}$) de la table de référence (LUT) et la ou les valeurs de réflectance mesurées ($R_{mesure}$).

8. Procédé selon la revendication 7, dans lequel la zone (ZS, ZC) de la peau (12) est éclairée via une fibre optique d'excitation (24), et la mesure du spectre est effectuée via une pluralité de fibres optiques de détection (26) reliées au spectromètre (16), les fibres de détection (26) étant à des distances différentes ($D_i$) de la fibre d'excitation (24), et dans lequel la prédétermination de la table de référence (LUT) est effectuée pour lesdites différentes distances ($D_i$), chaque valeur ($R_{LUT,Di}$) de ladite table étant prédéterminée pour un couple respectif de valeurs du coefficient d'absorption ($\mu_a$) et du coefficient de diffusion ($\mu_s$) et pour lesdites différentes distances ($D_i$), au moins une valeur ($R_{mesure,Di}$) de la réflectance de la zone à caractériser étant mesurée pour chacune desdites distance ($D_i$) et à l'aide du spectromètre (16) lors de l'étape de mesure (110).

9. Procédé selon l'une quelconque des revendications précédentes, dans lequel, lors de l'étape (120) de calcul de la concentration du ou des chromophores ($HbO_2$, Hb), la concentration du ou des chromophores est d'abord calculée pour une zone saine (ZS) de la peau (12) à partir de la ou des valeurs du coefficient d'absorption ($\mu_a$) pour ladite zone saine (ZS), et la concentration du ou des chromophores ($HbO_2$, Hb) est ensuite calculée pour la zone à caractériser (ZC) à partir de la concentration du ou des chromophores pour la zone saine (ZS) et de la ou des valeurs du coefficient d'absorption ($\mu_a$) pour ladite zone à caractériser (ZC).

**10.** Procédé selon l'une quelconque des revendications précédentes, dans lequel le ou chaque chromophore est choisi parmi le groupe constitué de : l'eau, la mélanine, l'oxyhémoglobine, la déoxyhémoglobine et la bilirubine, la concentration en oxyhémoglobine ($HbO_2$) et la concentration en déoxyhémoglobine (Hb) étant calculées de préférence.

**11.** Système (10) de calcul d'un indicateur (IND) de quantification d'une réaction dermique de la peau (12) d'un être vivant, telle qu'une réaction dermique suite à l'injection d'un principe actif, la peau (12) comportant une pluralité de chromophores ($HbO_2$, Hb, Mel, Bil), le système (10) comprenant :

    - une source de lumière (14) propre à émettre un faisceau lumineux d'excitation pour éclairer une zone à caractériser (ZC) de la peau (12), ladite zone à caractériser (ZC) comportant la réaction dermique,
    - un spectromètre (16) propre à mesurer le spectre (M) d'un rayonnement rétrodiffusé issu de la peau (12) suite à l'éclairement de ladite zone à caractériser (ZC),
    - une unité de traitement d'informations (18) comportant :

        + des premiers moyens (34) de détermination, à partir du spectre mesuré (M) et pour au moins une valeur donnée de la longueur d'onde ($\lambda$) du faisceau lumineux, d'une valeur du coefficient d'absorption ($\mu_a$) de la zone à caractériser (ZC),
        + des premiers moyens (38) de calcul pour calculer, à partir de la ou chaque valeur déterminée du coefficient d'absorption ($\mu_a$), la concentration d'au moins un chromophore ($HbO_2$, Hb) de la peau, et
        + des deuxièmes moyens (40) de calcul pour calculer l'indicateur de quantification de la réaction dermique (IND) en fonction de la concentration du ou de chaque chromophore ($HbO_2$, Hb) précédemment calculée,

    **caractérisé en ce que** les premiers moyens de détermination (34) sont en outre propres à déterminer, à partir du spectre mesuré (M) et pour au moins une valeur donnée de la longueur d'onde ($\lambda$) du faisceau lumineux, une valeur du coefficient de diffusion ($\mu_s$) de la zone à caractériser (ZC), et
    **en ce que** les deuxièmes moyens de calcul (40) sont propres à calculer l'indicateur de quantification de la réaction dermique (IND) en fonction également de la ou chaque valeur déterminée du coefficient de diffusion ($\mu_s$), l'indicateur de quantification de la réaction dermique (IND) vérifiant l'équation suivante :

$$IND = \alpha + \beta \times Ox + \gamma \times Deox + \delta \times Dif + \varepsilon \times Abs$$

    où IND représente l'indicateur de quantification de la réaction dermique,
    $\alpha$, $\beta$, $\gamma$, $\delta$ et $\varepsilon$ sont des coefficients prédéterminés pour une valeur donnée du délai entre l'instant de mesure du spectre d'un rayonnement rétrodiffusé et l'instant d'injection du principe actif, avec $\beta$, $\gamma$, $\delta$ et $\varepsilon$ de valeurs non nulles,
    Ox et Deox représentent la concentration en oxyhémoglobine ($HbO_2$) et respectivement en déoxyhémoglobine (Hb), pour la zone à caractériser (ZC),
    Dif et Abs représentent une moyenne, telle que la moyenne arithmétique, de la ou des valeurs déterminées du coefficient de diffusion ($\mu_s$) de la zone à caractériser (ZC), et respectivement de la ou des valeurs déterminées du coefficient d'absorption ($\mu_a$) de la zone à caractériser (ZC).

**12.** Système (10) de calcul d'un indicateur (IND) de quantification d'une réaction dermique de la peau (12) d'un être vivant, telle qu'une réaction dermique suite à l'injection d'un principe actif, la peau (12) comportant une pluralité de chromophores ($HbO_2$, Hb, Mel, Bil), le système (10) comprenant :

    - une source de lumière (14) propre à émettre un faisceau lumineux d'excitation pour éclairer une zone à caractériser (ZC) de la peau (12), ladite zone à caractériser (ZC) comportant la réaction dermique,
    - un spectromètre (16) propre à mesurer le spectre (M) d'un rayonnement rétrodiffusé issu de la peau (12) suite à l'éclairement de ladite zone à caractériser (ZC),
    - une unité de traitement d'informations (18) comportant :

        + des premiers moyens (34) de détermination, à partir du spectre mesuré (M) et pour au moins une valeur donnée de la longueur d'onde ($\lambda$) du faisceau lumineux, d'une valeur du coefficient d'absorption ($\mu_a$) de la zone à caractériser (ZC),
        + des premiers moyens (38) de calcul pour calculer, à partir de la ou chaque valeur déterminée du coefficient d'absorption ($\mu_a$), la concentration d'au moins un chromophore ($HbO_2$, Hb) de la peau, et

+ des deuxièmes moyens (40) de calcul pour calculer l'indicateur de quantification de la réaction dermique (IND) en fonction de la concentration du ou de chaque chromophore (HbO$_2$, Hb) précédemment calculée,

**caractérisé en ce que** les premiers moyens de détermination (34) sont en outre propres à déterminer, à partir du spectre mesuré (M) et pour au moins une valeur donnée de la longueur d'onde ($\lambda$) du faisceau lumineux, une valeur du coefficient de diffusion ($\mu_s$) de la zone à caractériser (ZC), et

**en ce que** les deuxièmes moyens de calcul (40) sont propres à calculer l'indicateur de quantification de la réaction dermique (IND) en fonction également de la ou chaque valeur déterminée du coefficient de diffusion ($\mu_s$), les premiers moyens de calcul (38 étant en outre adaptés pour calculer la concentration du ou des chromophores pour une zone saine (ZS) de la peau (12) à partir de la ou des valeurs du coefficient d'absorption ($\mu_a$) pour ladite zone saine (ZS), et

les deuxièmes moyens de calcul (40) étant en outre adaptés pour calculer l'indicateur de quantification de la réaction dermique (IND) en fonction d'un écart ($\Delta$Ox, $\Delta$Deox) entre les concentrations du ou de chaque chromophore (Hb, HbO$_2$) de la zone saine (ZS) et de la zone à caractériser (ZC), en fonction du ou des écarts ($\Delta$Dif) entre les valeurs déterminées du coefficient de diffusion ($\mu_s$) de la zone saine (ZS) et de la zone à caractériser (ZC), et en fonction en outre du ou des écarts ($\Delta$Abs) entre les valeurs déterminées du coefficient d'absorption ($\mu_a$) pour une zone saine (ZS) de la peau (12) et de la zone à caractériser (ZC), l'indicateur de quantification de la réaction dermique (IND) vérifiant l'équation suivante :

$$\text{IND} = \alpha' + \beta' \times \Delta\text{Ox} + \gamma' \times \Delta\text{Deox} + \delta' \times \Delta\text{Dif} + \varepsilon' \times \Delta\text{Abs}$$

où IND représente l'indicateur de quantification de la réaction dermique,
$\alpha'$, $\beta'$, $\gamma'$, $\delta'$ et $\varepsilon'$ sont des coefficients prédéterminés pour une valeur donnée du délai entre l'instant de mesure du spectre d'un rayonnement rétrodiffusé et l'instant d'injection du principe actif, avec $\beta'$, $\gamma'$, $\delta'$ et $\varepsilon'$ de valeurs non nulles,
$\Delta$Ox et $\Delta$Deox représentent un écart entre les concentrations en oxyhémoglobine (HbO$_2$) de la zone saine (ZS) et de la zone à caractériser (ZC), et respectivement un écart entre les concentrations en déoxyhémoglobine (Hb) de la zone saine (ZS) et de la zone à caractériser (ZC),
$\Delta$Dif et $\Delta$Abs représentent une moyenne, telle que la moyenne arithmétique, du ou des écarts entre les valeurs déterminées du coefficient de diffusion ($\mu_s$) de la zone saine (ZS) et de la zone à caractériser (ZC), et respectivement du ou des écarts entre les valeurs déterminées du coefficient d'absorption ($\mu_a$) de la zone saine (ZS) et de la zone à caractériser (ZC).

**Patentansprüche**

1. Verfahren zum Berechnen eines Quantifizierungsindikators (IND) einer dermalen Reaktion der Haut (12) eines Lebewesens, wie beispielsweise einer dermalen Reaktion nach der Injektion eines Wirkstoffs, die Haut (12) umfassend eine Vielzahl von Chromophoren (HbO$_2$, Hb, Mel, Bil),

das Verfahren umfassend die folgenden Schritte:

- Beleuchten (100) eines zu charakterisierenden Bereichs (ZC) der Haut (12) über einen Erregungslichtstrahl, der von einer Lichtquelle (14) emittiert wird, der zu charakterisierende Bereich (ZC) umfassend die Hautreaktion,
- Messen (110), mittels eines Spektrometers (16), des Spektrums (M) einer rückgestreuten Strahlung, die von der Haut (12) infolge der Beleuchtung des zu charakterisierenden Bereichs (ZC) ausgeht,
- Bestimmen (120), anhand des gemessenen Spektrums (M) und für mindestens einen gegebenen Wert der Wellenlänge ($\lambda$) des Lichtstrahls, eines Werts des Absorptionskoeffizienten ($\mu_a$) des zu charakterisierenden Bereichs,
- Berechnen (130), anhand des oder jedes bestimmten Werts des Absorptionskoeffizienten ($\mu_a$), der Konzentration (Ox, Deox) mindestens eines Chromophors (HbO$_2$, Hb) der Haut, und
- Berechnen (140) des Quantifizierungsindikators der Hautreaktion (IND) abhängig von der zuvor berechneten Konzentration des oder jedes Chromophors (Ox, Deox; $\Delta$Ox, $\Delta$Deox),

wobei das Verfahren **dadurch gekennzeichnet ist, dass** es ferner das Bestimmen (120), anhand des gemessenen Spektrums (M) und für mindestens einen gegebenen Wert der Wellenlänge ($\lambda$) des Lichtstrahls, eines

Werts des Diffusionskoeffizienten ($\mu_s$) des zu charakterisierenden Bereichs umfasst,
dass der Quantifizierungsindikator der dermalen Reaktion (IND) auch abhängig von dem oder jedem bestimmten Wert des Diffusionskoeffizienten ($\mu_s$, Dif; $\Delta$Dif) berechnet wird,
dass der Quantifizierungsindikator der dermalen Reaktion (IND) die folgende Gleichung erfüllt:

$$IND = \alpha + \beta \times Ox + \gamma \times Deox + \delta \times Dif + \varepsilon \times Abs$$

wobei IND den Quantifizierungsindikator der dermalen Reaktion darstellt,
$\alpha$, $\beta$, $\gamma$, $\delta$ und $\varepsilon$ vorbestimmte Koeffizienten für einen gegebenen Wert der Verzögerung zwischen dem Zeitpunkt der Messung des Spektrums einer rückgestreuten Strahlung und dem Zeitpunkt der Injektion des Wirkstoffs sind, wobei $\beta$, $\gamma$, $\delta$ und $\varepsilon$ von Null verschiedene Werte sind,
Ox und Deox die Konzentration von Oxyhämoglobin (HbO$_2$) und jeweils Deoxyhämoglobin (Hb) für den zu charakterisierenden Bereich (ZC) darstellen,
Dif und Abs einen Mittelwert, wie z. B. das arithmetische Mittel, des oder der bestimmten Werte des Diffusionskoeffizienten ($\mu_s$) des zu charakterisierenden Bereichs (ZC) und jeweils des oder der bestimmten Werte des Absorptionskoeffizienten ($\mu_a$) des zu charakterisierenden Bereichs (ZC) darstellen.

2. Verfahren zum Berechnen eines Quantifizierungsindikators (IND) einer dermalen Reaktion der Haut (12) eines Lebewesens, wie z. B. einer dermalen Reaktion nach der Injektion eines Wirkstoffs, wobei die Haut (12) eine Vielzahl von Chromophoren (HbO$_2$, Hb, Mel, Bil) umfasst,

das Verfahren umfassend die folgenden Schritte:

- Beleuchten (100) eines zu charakterisierenden Bereichs (ZC) der Haut (12) über einen Erregungslichtstrahl, der von einer Lichtquelle (14) emittiert wird, der zu charakterisierende Bereich (ZC) umfassend die Hautreaktion,
- Messen (110), mittels eines Spektrometers (16), des Spektrums (M) einer rückgestreuten Strahlung, die von der Haut (12) infolge der Beleuchtung des zu charakterisierenden Bereichs (ZC) ausgeht,
- Bestimmen (120), anhand des gemessenen Spektrums (M) und für mindestens einen gegebenen Wert der Wellenlänge ($\lambda$) des Lichtstrahls, eines Werts des Absorptionskoeffizienten ($\mu_a$) des zu charakterisierenden Bereichs,
- Berechnen (130), anhand des oder jedes bestimmten Werts des Absorptionskoeffizienten ($\mu_a$), der Konzentration (Ox, Deox) mindestens eines Chromophors (HbO$_2$, Hb) der Haut, und
- Berechnen (140) des Quantifizierungsindikators der Hautreaktion (IND) abhängig von der zuvor berechneten Konzentration des oder jedes Chromophors (Ox, Deox; $\Delta$Ox, $\Delta$Deox),

wobei das Verfahren **dadurch gekennzeichnet ist, dass** es ferner das Bestimmen (120), anhand des gemessenen Spektrums (M) und für mindestens einen gegebenen Wert der Wellenlänge ($\lambda$) des Lichtstrahls, eines Werts des Diffusionskoeffizienten ($\mu_s$) des zu charakterisierenden Bereichs umfasst,
dass der Quantifizierungsindikator der dermalen Reaktion (IND) auch abhängig von dem oder jedem bestimmten Wert des Diffusionskoeffizienten ($\mu_s$, Dif; $\Delta$Dif) berechnet wird,
wobei in Schritt (130) eines Berechnens der Konzentration des oder der Chromophore (HbO$_2$, Hb) die Konzentration des oder der Chromophore ferner für einen gesunden Bereich (ZS) der Haut (12) anhand des Werts oder der Werte des Absorptionskoeffizienten ($\mu_a$) für den gesunden Bereich (ZS) berechnet wird, und
in dem Berechnungsschritt (140) der Quantifizierungsindikator der Hautreaktion (IND) abhängig von einem Abstand ($\Delta$Ox, $\Delta$Deox) zwischen den Konzentrationen des oder jedes Chromophors (Hb, HbO$_2$) des gesunden Bereichs (ZS) und des zu charakterisierenden Bereichs (ZC) und abhängig von dem oder den Abständen ($\Delta$Dif) zwischen den bestimmten Werten des Diffusionskoeffizienten ($\mu_s$)des gesunden Bereichs (ZS) und des zu charakterisierenden Bereichs (ZC), und ferner abhängig von der oder den Abweichungen ($\Delta$Abs) zwischen den bestimmten Werten des Absorptionskoeffizienten ($\mu_a$) für einen gesunden Bereich (ZS) der Haut (12) und des zu charakterisierenden Bereichs (ZC) berechnet wird, und
der Quantifizierungsindikator der dermalen Reaktion (IND) die folgende Gleichung erfüllt:

$$IND = \alpha' + \beta' \times \Delta Ox + \gamma' \times \Delta Deox + \delta' \times \Delta Dif + \varepsilon' \times \Delta Abs$$

wobei IND den Quantifizierungsindikator der dermalen Reaktion darstellt,

$\alpha$', $\beta$', $\gamma$, $\delta$' und $\epsilon$' vorbestimmte Koeffizienten für einen gegebenen Wert der Verzögerung zwischen dem Zeitpunkt der Messung des Spektrums einer rückgestreuten Strahlung und dem Zeitpunkt der Injektion des Wirkstoffs sind, wobei $\beta$', $\gamma$', $\delta$' und $\epsilon$' von Null verschiedene Werte sind,

$\Delta$Ox und $\Delta$Deox eine Abweichung zwischen den Oxyhämoglobin-Konzentrationen (HbO$_2$) des gesunden Bereichs (ZS) und des zu charakterisierenden Bereichs (ZC) und jeweils eine Abweichung zwischen den Deoxyhämoglobin-Konzentrationen (Hb) des gesunden Bereichs (ZS) und des zu charakterisierenden Bereichs (ZC) darstellen,

$\Delta$Dif und $\Delta$Abs einen Mittelwert, wie z. B. das arithmetische Mittel, des oder der Abweichungen zwischen den bestimmten Werten des Diffusionskoeffizienten ($\mu_s$) des gesunden Bereichs (ZS) und des zu charakterisierenden Bereichs (ZC), und jeweils des oder der Abweichungen zwischen den bestimmten Werten des Absorptionskoeffizienten ($\mu_a$) des gesunden Bereichs (ZS) und des zu charakterisierenden Bereichs (ZC) darstellen.

3. Verfahren nach Anspruch 1 oder 2, wobei in dem Berechnungsschritt (140) der Quantifizierungsindikator der dermalen Reaktion (IND) zusätzlich abhängig von dem oder jedem bestimmten Wert des Absorptionskoeffizienten ($\mu_a$, Abs; $\Delta$Abs) berechnet wird.

4. Verfahren nach Anspruch 3, wobei der Quantifizierungsindikator der Hautreaktion (IND) abhängig von einem Mittelwert (Abs; $\Delta$Abs), z. B. des arithmetischen Mittels, einer Vielzahl von Werten des Absorptionskoeffizienten ($\mu_a$) berechnet wird, die für eine Vielzahl von Werten der Wellenlänge ($\lambda$) des Lichtstrahls zwischen 450 nm und 800 nm, vorzugsweise zwischen 450 nm und 700 nm, bevorzugter zwischen 500 nm und 650 nm, bestimmt werden.

5. Verfahren nach einem der vorherigen Ansprüche, wobei der Quantifizierungsindikator für die Hautreaktion (IND) abhängig von einem Mittelwert (Dif; $\Delta$Dif), wie z. B. des arithmetischen Mittelwerts, einer Vielzahl von Werten des Diffusionskoeffizienten ($\mu_s$) berechnet wird, die für eine Vielzahl von Werten der Wellenlänge ($\lambda$) des Lichtstrahls zwischen 450 nm und 800 nm, vorzugsweise zwischen 650 nm und 800 nm, weiter vorzugsweise zwischen 740 nm und 760 nm, bestimmt werden.

6. Verfahren nach einem der vorherigen Ansprüche, wobei der Bereich (ZS, ZC) der Haut (12) über eine optische Erregungsfaser (24) beleuchtet wird und die Messung des Spektrums über eine Vielzahl von mit dem Spektrometer (16) verbundenen optischen Detektionsfasern (26) durchgeführt wird, wobei die Detektionsfasern (26) in unterschiedlichen Abständen (D$_i$) von der Erregungsfaser (24) sind, und wobei die Bestimmung, für mindestens einen gegebenen Wert der Wellenlänge ($\lambda$) des Lichtstrahls, des Werts des Absorptionskoeffizienten ($\mu_a$) bzw. des Diffusionskoeffizienten ($\mu_s$), abhängig von den Spektren (M), die für die verschiedenen Abstände (D$_i$) gemessen werden, durchgeführt wird.

7. Verfahren nach einem der vorherigen Ansprüche, wobei das Verfahren ferner das Vorbestimmen einer Referenztabelle (LUT) umfassend eine Vielzahl von Werten (R$_{LUT}$) für das Reflexionsvermögen der Haut, wobei jeder Wert (R$_{LUT}$) der Tabelle für ein jeweiliges Paar von Werten des Absorptionskoeffizienten ($\mu_a$) und des Streukoeffizienten ($\mu_s$) vorbestimmt ist, wobei mindestens ein Wert (R$_{Messung}$) des Reflexionsvermögens des zu charakterisierenden Bereichs mittels des Spektrometers (16) in dem Messschritt (110) gemessen wird, und wobei in dem Schritt (120) eines Bestimmens des Absorptionskoeffizienten ($\mu_a$) und des Diffusionskoeffizienten ($\mu_s$) das Paar von bestimmten Werten des Absorptionskoeffizienten ($\mu_a$) und des Diffusionskoeffizienten ($\mu_s$) jenes ist, das den Fehler zwischen den vorbestimmten Reflexionsgraden (R$_{LUT}$) der Referenztabelle (LUT) und dem gemessenen Reflexionsgradwert oder den gemessenen Reflexionsgradwerten $_($R$_{Messung})$- minimiert.

8. Verfahren nach Anspruch 7, wobei der Bereich (ZS, ZC) der Haut (12) über eine optische Erregungsfaser (24) beleuchtet wird und die Messung des Spektrums über eine Vielzahl von mit dem Spektrometer (16) verbundenen optischen Detektionsfasern (26) durchgeführt wird, wobei die Detektionsfasern (26) in unterschiedlichen Abständen (D$_i$) von der Erregungsfaser (24) sind, und wobei die Vorbestimmung der Referenztabelle (LUT) für die verschiedenen Abstände (D$_i$) durchgeführt wird, wobei jeder Wert (R$_{LUT,Di}$) der Tabelle für ein jeweiliges Paar von Werten des Absorptionskoeffizienten ($\mu_a$) und des Diffusionskoeffizienten ($\mu_s$) und für die verschiedenen Abstände (D$_i$) vorbestimmt ist, wobei mindestens ein Wert (R$_{Messung,Di}$) des Reflexionsvermögens des zu charakterisierenden Bereichs für jeden der Abstände (D$_i$) und mittels des Spektrometers (16) während des Messschritts (110) gemessen wird.

9. Verfahren nach einem der vorherigen Ansprüche, wobei in dem Schritt (120) eines Berechnens der Konzentration des oder der Chromophore (HbO$_2$, Hb) die Konzentration des oder der Chromophore zunächst für einen gesunden Bereich (ZS) der Haut (12) anhand des Werts oder der Werte des Absorptionskoeffizienten ($\mu_a$) für den gesunden

Bereich (ZS) berechnet wird, und die Konzentration des oder der Chromophore (HbO$_2$, Hb) wird dann für den zu charakterisierenden Bereich (ZC) anhand der Konzentration des oder der Chromophore für den gesunden Bereich (ZS) und dem oder den Werten des Absorptionskoeffizienten (μ$_a$) für den zu charakterisierenden Bereich (ZC) berechnet.

10. Verfahren nach einem der vorherigen Ansprüche, wobei der oder jeder Chromophor ausgewählt ist aus der Gruppe, bestehend aus: Wasser, Melanin, Oxyhämoglobin, Deoxyhämoglobin und Bilirubin, wobei vorzugsweise die Oxyhämoglobin-Konzentration (HbO$_2$) und die Deoxyhämoglobin-Konzentration (Hb) berechnet werden.

11. System (10) zum Berechnen eines Indikators (IND) zur Quantifizierung einer dermalen Reaktion der Haut (12) eines Lebewesens, wie z. B. einer dermalen Reaktion nach der Injektion eines Wirkstoffs, wobei die Haut (12) eine Vielzahl von Chromophoren (HbO$_2$, Hb, Mel, Bil) aufweist, das System (10) umfassend:

- eine Lichtquelle (14), die geeignet ist, um einen Anregungslichtstrahl zu emittieren, um einen zu charakterisierenden Bereich (ZC) der Haut (12) zu beleuchten, wobei der zu charakterisierende Bereich (ZC) die dermale Reaktion umfasst,
- ein Spektrometer (16), das geeignet ist, um das Spektrum (M) einer Rückstreustrahlung zu messen, die von der Haut (12) infolge der Beleuchtung des zu charakterisierenden Bereichs (ZC) ausgeht,
- eine Datenverarbeitungseinheit (18), umfassend:

+ erste Bestimmungseinrichtungen (34) zur Bestimmung, anhand des gemessenen Spektrums (M) und für mindestens einen gegebenen Wert der Wellenlänge (λ) des Lichtstrahls, eines Werts des Absorptionskoeffizienten (μ$_a$) des zu charakterisierenden Bereichs (ZC),
+ erste Recheneinrichtungen (38), um anhand des oder jedes bestimmten Werts des Absorptionskoeffizienten (μ$_a$) die Konzentration von mindestens einem Chromophor (HbO$_2$, Hb) der Haut zu berechnen, und
+ zweite Recheneinrichtung (40), um den Quantifizierungsindikator (IND) für die Hautreaktion abhängig von der zuvor berechneten Konzentration des oder jedes Chromophors (HbO$_2$, Hb) zu berechnen,

**dadurch gekennzeichnet, dass** die ersten Bestimmungseinrichtungen (34) ferner geeignet sind, anhand des gemessenen Spektrums (M) und für mindestens einen gegebenen Wert der Wellenlänge (λ) des Lichtstrahls einen Wert des Diffusionskoeffizienten (μ$_s$) des zu charakterisierenden Bereichs (ZC) zu bestimmen, und dass die zweiten Berechnungsmittel (40) geeignet sind, um den Quantifizierungsindikator der dermalen Reaktion (IND) auch abhängig von dem oder jedem bestimmten Wert des Diffusionskoeffizienten (μ$_s$) zu berechnen, wobei der Quantifizierungsindikator der dermalen Reaktion (IND) die folgende Gleichung erfüllt:

$$IND = \alpha + \beta \times Ox + \gamma \times Deox + \delta \times Dif + \varepsilon \times Abs$$

wobei IND den Quantifizierungsindikator der dermalen Reaktion darstellt,
α, β, γ, δ und ε vorbestimmte Koeffizienten für einen gegebenen Wert der Verzögerung zwischen dem Zeitpunkt der Messung des Spektrums einer rückgestreuten Strahlung und dem Zeitpunkt der Injektion des Wirkstoffs sind, wobei β, γ, δ und ε von Null verschiedene Werte sind,
Ox und Deox die Konzentration von Oxyhämoglobin (HbO$_2$) und jeweils Deoxyhämoglobin (Hb) für den zu charakterisierenden Bereich (ZC) darstellen,
Dif und Abs einen Mittelwert, wie z. B. das arithmetische Mittel, des oder der bestimmten Werte des Diffusionskoeffizienten (μ$_s$) des zu charakterisierenden Bereichs (ZC) und jeweils des oder der bestimmten Werte des Absorptionskoeffizienten (μ$_a$) des zu charakterisierenden Bereichs (ZC) darstellen.

12. System (10) zum Berechnen eines Indikators (IND) zur Quantifizierung einer dermalen Reaktion der Haut (12) eines Lebewesens, wie z. B. einer dermalen Reaktion nach der Injektion eines Wirkstoffs, wobei die Haut (12) eine Vielzahl von Chromophoren (HbO$_2$, Hb, Mel, Bil) aufweist, das System (10) umfassend:

- eine Lichtquelle (14), die geeignet ist, um einen Anregungslichtstrahl zu emittieren, um einen zu charakterisierenden Bereich (ZC) der Haut (12) zu beleuchten, wobei der zu charakterisierende Bereich (ZC) die dermale Reaktion umfasst,
- ein Spektrometer (16), das geeignet ist, um das Spektrum (M) einer Rückstreustrahlung zu messen, die von der Haut (12) infolge der Beleuchtung des zu charakterisierenden Bereichs (ZC) ausgeht,

- eine Datenverarbeitungseinheit (18), umfassend:

+ erste Bestimmungseinrichtungen (34) zur Bestimmung, anhand des gemessenen Spektrums (M) und für mindestens einen gegebenen Wert der Wellenlänge ($\lambda$) des Lichtstrahls, eines Werts des Absorptionskoeffizienten ($\mu_a$) des zu charakterisierenden Bereichs (ZC),
+ erste Recheneinrichtungen (38), um anhand des oder jedes bestimmten Werts des Absorptionskoeffizienten ($\mu_a$) die Konzentration von mindestens einem Chromophor (HbO$_2$, Hb) der Haut zu berechnen, und
+ zweite Recheneinrichtung (40), um den Quantifizierungsindikator (IND) für die Hautreaktion abhängig von der zuvor berechneten Konzentration des oder jedes Chromophors (HbO$_2$, Hb) zu berechnen,

**dadurch gekennzeichnet, dass** die ersten Bestimmungseinrichtungen (34) ferner geeignet sind, anhand des gemessenen Spektrums (M) und für mindestens einen gegebenen Wert der Wellenlänge ($\lambda$) des Lichtstrahls einen Wert des Diffusionskoeffizienten ($\mu_s$) des zu charakterisierenden Bereichs (ZC) zu bestimmen, und dass die zweiten Berechnungsmittel (40) geeignet sind, um den Quantifizierungsindikator der dermalen Reaktion (IND) auch abhängig von dem oder jedem bestimmten Wert des Diffusionskoeffizienten ($\mu_s$) zu berechnen, wobei die ersten Berechnungseinrichtungen (38) ferner geeignet sind, um die Konzentration des oder der Chromophore für einen gesunden Bereich (ZS) der Haut (12) anhand des oder der Werte des Absorptionskoeffizienten ($\mu_a$) für den gesunden Bereich (ZS) zu berechnen, und wobei die zweiten Berechnungsmittel (40) ferner geeignet sind, um den Quantifizierungsindikator der Hautreaktion (IND) abhängig von einem Abstand ($\Delta$Ox, $\Delta$Deox) zwischen den Konzentrationen des oder jedes Chromophors (Hb, HbO$_2$) des gesunden Bereichs (ZS) und des zu charakterisierenden Bereichs (ZC) und abhängig von dem oder den Abständen ($\Delta$Dif) zwischen den bestimmten Werten des Diffusionskoeffizienten ($\mu_s$) des gesunden Bereichs (ZS) und des zu charakterisierenden Bereichs (ZC), und ferner abhängig von der oder den Abweichungen ($\Delta$Abs) zwischen den bestimmten Werten des Absorptionskoeffizienten ($\mu_a$) für einen gesunden Bereich (ZS) der Haut (12) und des zu charakterisierenden Bereichs (ZC) zu berechnen, und wobei der Quantifizierungsindikator der dermalen Reaktion (IND) die folgende Gleichung erfüllt:

$$IND = \alpha' + \beta' \times \Delta Ox + \gamma' \times \Delta Deox + \delta' \times \Delta Dif + \varepsilon' \times \Delta Abs$$

wobei IND den Quantifizierungsindikator der dermalen Reaktion darstellt, $\alpha'$, $\beta'$, $\gamma$, $\delta'$ und $\varepsilon'$ vorbestimmte Koeffizienten für einen gegebenen Wert der Verzögerung zwischen dem Zeitpunkt der Messung des Spektrums einer rückgestreuten Strahlung und dem Zeitpunkt der Injektion des Wirkstoffs sind, wobei $\beta'$, $\gamma'$, $\delta'$ und $\varepsilon'$ von Null verschiedene Werte sind,
$\Delta$Ox und $\Delta$Deox eine Abweichung zwischen den Oxyhämoglobin-Konzentrationen (HbO$_2$) des gesunden Bereichs (ZS) und des zu charakterisierenden Bereichs (ZC) und jeweils eine Abweichung zwischen den Deoxyhämoglobin-Konzentrationen (Hb) des gesunden Bereichs (ZS) und des zu charakterisierenden Bereichs (ZC) darstellen,
$\Delta$Dif und $\Delta$Abs einen Mittelwert, wie z. B. das arithmetische Mittel, des oder der Abweichungen zwischen den bestimmten Werten des Diffusionskoeffizienten ($\mu_s$) des gesunden Bereichs (ZS) und des zu charakterisierenden Bereichs (ZC), und jeweils des oder der Abweichungen zwischen den bestimmten Werten des Absorptionskoeffizienten ($\mu_a$) des gesunden Bereichs (ZS) und des zu charakterisierenden Bereichs (ZC) darstellen.

## Claims

1. A method for calculating a quantification indicator (IND) for quantifying a dermal reaction on the skin (12) of a living being, such as a dermal reaction following the injection of an active ingredient, the skin (12) having a plurality of chromophores (HbO$_2$, Hb, Mel, Bil),

the method comprising the following steps :

- the illumination (100) of a zone to be characterised (ZC) on the skin (12) via an excitation light beam emitted by a source of light (14), the skin reaction being included in the zone to be characterised a zone to be characterised (ZC),
- the measurement (110), by means of a spectrometer (16), of the spectrum (M) of a back scattered radiation coming from the skin (12) as a result of the illumination of the said zone to be characterised (ZC),
- the determination (120), on the basis of the measured spectrum (M) and for at least one given value of

the wave length ($\lambda$) of the light beam, of a value for the absorption coefficient ($\mu_a$) of the zone to be characterised,

- the calculation (130), on the basis of the or each determined value of the absorption coefficient ($\mu_a$), of the concentration (Ox, Deox) of at least one chromophore ($HbO_2$, Hb) in the skin, and
- the calculation (140), of the indicator for quantification of the dermal reaction (IND) on the basis of the previously calculated concentration of the or each chromophore (Ox, Deox ; $\Delta$Ox, $\Delta$Deox),

the method being **characterised in that** it further comprises of the determination (120), on the basis of the measured spectrum (M) and for at least one given value of the wave length ($\lambda$) of the light beam, of a value for the diffusion coefficient ($\mu_s$) of the zone to be **characterised,**

**in that** the indicator for quantification of the dermal reaction (IND) is also calculated on the basis of the or each determined value of the diffusion coefficient ($\mu_s$, Dif ; $\Delta$Dif),

**in that** the indicator for quantification of the dermal reaction (IND) satisfies the following equation :

$$IND = \alpha + \beta \times Ox + \gamma \times Deox + \delta \times Dif + \varepsilon \times Abs$$

where IND represents the indicator for quantification of the dermal reaction,

$\alpha$, $\beta$, $\gamma$, $\delta$ and $\varepsilon$ are predetermined coefficients for a given value of the time period between the moment of measurement of the spectrum of a back scattered radiation and the moment of injection of the active ingredient, with $\beta$, $\gamma$, $\delta$ and $\varepsilon$ having non-null values,

Ox and Deox represent the oxyhemoglobin concentration ($HbO_2$) and deoxyhemoglobin concentration (Hb), respectively, for the zone to be characterised (ZC),

Dif and Abs represent an average value, such as the arithmetic mean, respectively of the determined value or values of the diffusion coefficient ($\mu_s$) of the zone to be characterised (ZC), and of the determined value or values of the absorption coefficient ($\mu_a$) of the zone to be characterised (ZC).

2. A method for calculating a quantification indicator (IND) for quantifying a dermal reaction on the skin (12) of a living being, such as a dermal reaction following the injection of an active ingredient, the skin (12) having a plurality of chromophores ($HbO_2$, Hb, Mel, Bil),

the method comprising the following steps :

- the illumination (100) of a zone to be characterised (ZC) on the skin (12) via an excitation light beam emitted by a source of light (14), the skin reaction being included in the zone to be characterised a zone to be characterised (ZC),
- the measurement (110), by means of a spectrometer (16), of the spectrum (M) of a back scattered radiation coming from the skin (12) as a result of the illumination of the said zone to be characterised (ZC),
- the determination (120), on the basis of the measured spectrum (M) and for at least one given value of the wave length ($\lambda$) of the light beam, of a value for the absorption coefficient ($\mu_a$) of the zone to be characterised,
- the calculation (130), on the basis of the or each determined value of the absorption coefficient ($\mu_a$), of the concentration (Ox, Deox) of at least one chromophore ($HbO_2$, Hb) in the skin, and
- the calculation (140), of the indicator for quantification of the dermal reaction (IND) on the basis of the previously calculated concentration of the or each chromophore (Ox, Deox ; $\Delta$Ox, $\Delta$Deox),

the method being **characterised in that** it further comprises of the determination (120), on the basis of the measured spectrum (M) and for at least one given value of the wave length ($\lambda$) of the light beam, of a value for the diffusion coefficient ($\mu_s$) of the zone to be **characterised,**

**in that** the indicator for quantification of the dermal reaction (IND) is also calculated on the basis of the or each determined value of the diffusion coefficient ($\mu_s$, Dif ; $\Delta$Dif),

during the step (120) of calculation of the concentration of the chromophore or chromophores ($HbO_2$, Hb), the concentration of the chromophore or chromophores is also calculated for a healthy zone (ZS) of the skin (12) on the basis of the value or values of the absorption coefficient ($\mu_a$) for the said healthy zone (ZS), and

during the step of calculation (140), the indicator for quantification of the dermal reaction (IND) is calculated on the basis of a difference ($\Delta$Ox, $\Delta$Deox) between the concentrations of the or each chromophore (Hb, $HbO_2$) of the healthy zone (ZS) and of the zone to be characterised (ZC) and on the basis of the difference or differences ($\Delta$Dif) between the determined values of the diffusion coefficient ($\mu_s$) of the healthy zone (ZS) and the zone to be characterised (ZC), and further on the basis of the difference or differences ($\Delta$Abs) between the determined values of the absorption coefficient ($\mu_a$) of the healthy zone (ZS) and the zone to be characterised (ZC), and the indicator for quantification of the dermal reaction (IND) satisfies the following equation :

$$IND = \alpha' + \beta' \times \Delta Ox + \gamma' \times \Delta Deox + \delta' \times \Delta Dif + \varepsilon' \times \Delta Abs$$

where IND represents the indicator for quantification of the dermal reaction,

$\alpha'$, $\beta'$, $\gamma'$, $\delta'$ and $\varepsilon'$ are predetermined coefficients for a given value of the time period between the moment of measurement of the spectrum of a back scattered radiation and the moment of injection of the active ingredient, with $\beta'$, $\gamma'$, $\delta'$ and $\varepsilon'$ having non-null values,

$\Delta$Ox and $\Delta$Deox represent respectively, a difference between the oxyhemoglobin concentrations (HbO$_2$) of the healthy zone (ZS) and the zone to be characterised (ZC), and a difference between the deoxyhemoglobin concentrations (Hb) of the healthy zone (ZS) and the zone to be characterised (ZC),

$\Delta$Dif and $\Delta$Abs represent an average value, such as the arithmetic mean, respectively, of the difference or differences between the determined values of the diffusion coefficient ($\mu_s$) of the healthy zone (ZS) and the zone to be characterised (ZC), and of the difference or differences between the determined values of the absorption coefficient ($\mu_a$) of the healthy zone (ZS) and the zone to be characterised (ZC).

3. A method according to claim 1 or 2, wherein, during the step of calculation (140), the indicator for quantification of the dermal reaction (IND) is calculated furthermore on the basis of the or each determined value of the absorption coefficient ($\mu_a$, Abs ; $\Delta$Abs).

4. A method according to claim 3, wherein the indicator for quantification of the dermal reaction (IND) is calculated on the basis of an average value (Abs ; $\Delta$Abs), such as the arithmetic average, of a plurality of values of the absorption coefficient ($\mu_a$), determined for a plurality of values of the wave length ($\lambda$) of the light beam comprised between 450 nm and 800 nm, preferably between 450 nm and 700 nm, and more preferably between 500 nm and 650 nm.

5. A method according to any one of the preceding claims, wherein the indicator for quantification of the dermal reaction (IND) is calculated on the basis of an average value (Dif ; $\Delta$Dif), such as the arithmetic average, of a plurality of values of the diffusion coefficient ($\mu_s$), determined for a plurality of values of the wave length ($\lambda$) of the light beam comprised between 450 nm and 800 nm, preferably between 650 nm and 800 nm, and more preferably between 740 nm and 760 nm.

6. A method according to any one of the preceding claims, wherein the zone (ZS, ZC) of the skin (12) is illuminated via an excitation optical fibre (24), and measurement of the spectrum is performed via a plurality of detection optical fibres (26) connected to the spectrometer (16), the detection fibres (26) being at different distances (Di) from the excitation fibre (24), and

wherein determining, for at least one given value of the wave length ($\lambda$) of the light beam, the value of the determination, for at least one given value of the wave length of the light beam, of the value of the absorption coefficient ($\mu_a$), and the diffusion coefficient ($\mu_s$), respectively, is performed on the basis of the measured spectra (M) for the said different distances (Di).

7. A method according to any one of the preceding claims wherein the method further comprises the predetermination of a Look Up reference table (LUT) including a plurality of values (R$_{LUT}$) of the reflectance of the skin, each value (R$_{LUT}$) of the said table being predetermined for a respective pair of values of the absorption coefficient ($\mu_a$) and the diffusion coefficient ($\mu_s$), in which at least one value (R$_{measurement}$) of the reflectance of the zone to be characterised is measured with the use of the spectrometer (16) during the step of measurement (110), and during the step of determination (120) of the absorption coefficient ($\mu_a$) and the diffusion coefficient ($\mu_s$), the pair of determined values of the absorption coefficient ($\mu_a$) and the diffusion coefficient ($\mu_s$) is that which minimises the error between the predetermined reflectance values (R$_{LUT}$) of the reference table (LUT) and the measured reflectance value or values (R$_{measurement}$).

8. A method according to claim 7, wherein the zone (ZS, ZC) of the skin (12) is illuminated via an excitation optical fibre (24), and measurement of the spectrum is performed via a plurality of detection optical fibres (26) connected to the spectrometer (16), the detection fibres (26) being at different distances (D$_i$) from the excitation fibre (24), and wherein the predetermination of the reference table (LUT) is carried out for the said different distances (D$_i$), each value (R$_{LUT,Di}$) of the said table being predetermined for a respective pair of values of the absorption coefficient ($\mu_a$)

and the diffusion coefficient ($\mu_s$) and for the said different distances ($D_i$), at least one value ($R_{measurement,Di}$) of the reflectance of the zone to be characterised being measured for each of the said distances ($D_i$) and with the use of the spectrometer (16) during the measurement step (110).

9. A method according to any one of the preceding claims, wherein, during the step (120) of calculation of the concentration of the chromophore or chromophores (HbO$_2$, Hb), the concentration of the chromophore or chromophores is firstly calculated for a healthy zone (ZS) of the skin (12) on the basis of the value or values of the absorption coefficient ($\mu_a$) for the said healthy zone (ZS), and the concentration of the chromophore or chromophores (HbO$_2$, Hb) is then calculated for the zone to be characterised (ZC) on the basis of the concentration of the chromophore or chromophores for the said healthy zone (ZS) and of the value or values of the absorption coefficient ($\mu_a$) for the said zone to be characterised (ZC).

10. A method according to any one of the preceding claims, wherein the or each chromophore is selected from the group consisting of: water, melanin, oxyhemoglobin, deoxyhemoglobin and bilirubin,
the oxyhemoglobin concentration (HbO$_2$) and the deoxyhemoglobin concentration (Hb) are preferably calculated.

11. A system (10) for calculation of an indicator (IND) for quantification of the dermal reaction on the skin (12) of a living being, such as a dermal reaction following the injection of an active ingredient, the skin (12) having a plurality of chromophores (HbO$_2$, Hb, Mel, Bil), the system (10) comprising of the following :

- a light source (14) capable of emitting an excitation light beam in order to illuminate a zone to be characterised (ZC) on the skin (12), the skin reaction being included in the said zone to be characterised (ZC),
- a spectrometer (16) capable of measuring the spectrum (M) of a back scattered radiation coming from the skin (12) as a result of the illumination of the said zone to be characterised (ZC),
- an information processing unit (18) comprising of:

+ first determination means (34) for determining, on the basis of the measured spectrum (M) and for at least one given value of the wave length ($\lambda$) of the light beam, a value for the absorption coefficient ($\mu_a$) of the zone to be characterised (ZC),
+ first calculation means (38) for calculating, on the basis of the or each determined value of the absorption coefficient ($\mu_a$), the concentration of at least one chromophore (HbO$_2$, Hb) in the skin, and
+ second calculation means (40) for calculating the indicator for quantification of the dermal reaction (IND) on the basis of the previously calculated concentration of the or each chromophore (HbO$_2$, Hb),

**characterised in that** first determination means (34) are further capable of determining, on the basis of the measured spectrum (M) and for at least one given value of the wave length ($\lambda$) of the light beam, a value for the absorption coefficient ($\mu_s$) of the zone to be characterised (ZC), and
**in that** the second calculation means (40) are capable of calculating the indicator for quantification of the dermal reaction (IND) also on the basis of the or each determined value of the diffusion coefficient ($\mu_s$), the indicator for quantification of the dermal reaction (IND) satisfying the following equation :

$$\text{IND} = \alpha + \beta \times \text{Ox} + \gamma \times \text{Deox} + \delta \times \text{Dif} + \varepsilon \times \text{Abs}$$

where IND represents the indicator for quantification of the dermal reaction,
$\alpha$, $\beta$, $\gamma$, $\delta$ and $\varepsilon$ are predetermined coefficients for a given value of the time period between the moment of measurement of the spectrum of a back scattered radiation and the moment of injection of the active ingredient, with $\beta$, $\gamma$, $\delta$ and $\varepsilon$ having non-null values,
Ox and Deox represent the oxyhemoglobin concentration (HbO$_2$) and deoxyhemoglobin concentration (Hb), respectively, for the zone to be characterised (ZC),
Dif and Abs represent an average value, such as the arithmetic mean, respectively of the determined value or values of the diffusion coefficient ($\mu_s$) of the zone to be characterised (ZC), and of the determined value or values of the absorption coefficient ($\mu_a$) of the zone to be characterised (ZC).

12. A system (10) for calculation of an indicator (IND) for quantification of the dermal reaction on the skin (12) of a living being, such as a dermal reaction following the injection of an active ingredient, the skin (12) having a plurality of chromophores (HbO$_2$, Hb, Mel, Bil), the system (10) comprising of the following :

- a light source (14) capable of emitting an excitation light beam in order to illuminate a zone to be characterised (ZC) on the skin (12), the skin reaction being included in the said zone to be characterised (ZC),
- a spectrometer (16) capable of measuring the spectrum (M) of a back scattered radiation coming from the skin (12) as a result of the illumination of the said zone to be characterised (ZC),
- an information processing unit (18) comprising of:

+ first determination means (34) for determining, on the basis of the measured spectrum (M) and for at least one given value of the wave length ($\lambda$) of the light beam, a value for the absorption coefficient ($\mu_a$) of the zone to be characterised (ZC),
+ first calculation means (38) for calculating, on the basis of the or each determined value of the absorption coefficient ($\mu_a$), the concentration of at least one chromophore ($HbO_2$, Hb) in the skin, and
+ second calculation means (40) for calculating the indicator for quantification of the dermal reaction (IND) on the basis of the previously calculated concentration of the or each chromophore ($HbO_2$, Hb),

**characterised in that** first determination means (34) are further capable of determining, on the basis of the measured spectrum (M) and for at least one given value of the wave length ($\lambda$) of the light beam, a value for the absorption coefficient ($\mu_s$) of the zone to be characterised (ZC), and
**in that** the second calculation means (40) are capable of calculating the indicator for quantification of the dermal reaction (IND) also on the basis of the or each determined value of the diffusion coefficient ($\mu_s$),
the first calculation means (38) being further adapted for calculating the concentration of the chromophore or chromophores ($HbO_2$, Hb) for a healthy zone (ZS) of the skin (12) on the basis of the value or values of the absorption coefficient ($\mu_a$) for the said healthy zone (ZS), and
the second calculation means (40) being further adapted for calculating the indicator for quantification of the dermal reaction (IND) on the basis of a difference ($\Delta Ox$, $\Delta Deox$) between the concentrations of the or each chromophore (Hb, $HbO_2$) of the healthy zone (ZS) and of the zone to be characterised (ZC) and on the basis of the difference or differences ($\Delta Dif$) between the determined values of the diffusion coefficient ($\mu_s$) of the healthy zone (ZS) and the zone to be characterised (ZC), and further on the basis of the difference or differences ($\Delta Abs$) between the determined values of the absorption coefficient ($\mu_a$) of the healthy zone (ZS) and the zone to be characterised (ZC), the indicator for quantification of the dermal reaction (IND) satisfying the following equation :

$$IND = \alpha' + \beta' \times \Delta Ox + \gamma' \times \Delta Deox + \delta' \times \Delta Dif + \varepsilon' \times \Delta Abs$$

where IND represents the indicator for quantification of the dermal reaction,
$\alpha'$, $\beta'$, $\gamma'$, $\delta'$ and $\varepsilon'$ are predetermined coefficients for a given value of the time period between the moment of measurement of the spectrum of a back scattered radiation and the moment of injection of the active ingredient, with $\beta'$, $\gamma'$, $\delta'$ and $\varepsilon'$ having non-null values,
$\Delta Ox$ and $\Delta Deox$ represent respectively, a difference between the oxyhemoglobin concentrations ($HbO_2$) of the healthy zone (ZS) and the zone to be characterised (ZC), and a difference between the deoxyhemoglobin concentrations (Hb) of the healthy zone (ZS) and the zone to be characterised (ZC),
$\Delta Dif$ and $\Delta Abs$ represent an average value, such as the arithmetic mean, respectively, of the difference or differences between the determined values of the diffusion coefficient ($\mu_s$) of the healthy zone (ZS) and the zone to be characterised (ZC), and of the difference or differences between the determined values of the absorption coefficient ($\mu_a$) of the healthy zone (ZS) and the zone to be characterised (ZC).

Fig. 1

Fig. 2

Eclairement d'une zone de la peau comportant
une réaction dermique à quantifier — 100

Mesure du spectre du rayonnement rétrodiffusé — 110

Détermination de $\mu_a$ et $\mu_s$ pour au moins
une valeur de la longueur d'onde du faisceau d'excitation — 120

Calcul de la concentration d'au moins
un chromophore de la peau — 130

Calcul d'un indicateur de quantification
de la réaction dermique en fonction de
la concentration du ou de chaque chromophore
calculée et de la ou des valeurs de $\mu_s$ — 140

*Fig. 3*

*Fig. 4*

Fig. 5

Fig. 6

Fig. 7 (Art Antérieur)

Fig. 8

Fig. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2011124987 A1
- US 2012130257 A1
- US 2010160754 A1

**Littérature non-brevet citée dans la description**

- **KOLLIAS et al.** Interpreting diffuse réflectance for in vivo skin reactions in terms of chromophores. *Journal of Biophotonics en,* 2010
- **FARRELL et al.** diffusion theory model of spatially resolved, steady-state diffuse réflectance for the non-invasive détermination of tissue optical properties in vivo. *Medical Physics,* 1992